(19) Europäisches Patentamt
European Patent Office
Office européen des brevets

(11) EP 1 140 923 B1

# (12) FASCICULE DE BREVET EUROPEEN

(45) Date de publication et mention de la délivrance du brevet:
**05.03.2003 Bulletin 2003/10**

(21) Numéro de dépôt: **99958317.2**

(22) Date de dépôt: **14.12.1999**

(51) Int Cl.[7]: **C07D 413/06**, C07D 265/30

(86) Numéro de dépôt international:
**PCT/FR99/03123**

(87) Numéro de publication internationale:
**WO 00/039126 (06.07.2000 Gazette 2000/27)**

## (54) PROCEDE DE PREPARATION DU (R)- (+)-3- 1- 2- (4-BENZOYL-2-(3,4-DIFLUOROPHENYL)MORPHOLIN-2- YL)ETHYL]-4-PHENYLPIPERIDIN-4- YL -1,1-DIMETHYLUREE, DE SES SELS, SOLVATS ET/OU HYDRATES

VERFAHREN ZUR HERSTELLUNG VOM (R)-(+)-3-1-[2-(4-BENZOYL-2-(3,4-DIFLUOROPHENYL)MORPHOLIN-2-YL)ETHYL]-4-PHENYLPIPERIDIN-4-YL -1,1-DIMETHYLHARNSTOFF, DEREN SALZEN, SOLVATEN UND/ODER HYDRATEN

METHOD FOR PREPARING (R)- (+) -3 1- 2-( 4-BENZOYL- 2-(3,4-DIFLUOROPHENYL)MORPHOLIN- 2-YL)ETHYL]- 4-PHENYLPIPERIDIN -4-YL -1,1-DIMETHYLUREA, ITS SALTS SOLVATES AND/OR HYDRATES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **23.12.1998 FR 9816410**

(43) Date de publication de la demande:
**10.10.2001 Bulletin 2001/41**

(73) Titulaire: **SANOFI-SYNTHELABO**
**75013 Paris (FR)**

(72) Inventeurs:
- **AULOMBARD, Alain**
  **F-34970 Lattes (FR)**
- **BERNON, Françoise**
  **F-34980 Saint Clément la Rivière (FR)**
- **BONNEFOY, Sabrina**
  **F-34560 Montbazin (FR)**
- **BURGOS, Alain**
  **Exton, PA 19341 (US)**
- **CABOS, Claude**
  **F-34990 Juvignac (FR)**
- **LUCAS, Eric**
  **F-34150 La Boissière (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 673 928**
**WO-A-96/23787**
**WO-A-97/10211**

EP 1 140 923 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un nouveau procédé de préparation du (R)-(+)-3-{1-[2-(4-benzoyl-2-(3,4-difluorophényl)morpholin-2-yl)éthyl]-4-phénylpipéridin-4-yl}-1,1-diméthylurée, de ses sels, solvats et/ou hydrates.

Le (R)-(+)-3-{1-[2-(4-benzoyl-2-(3,4-difluorophényl) morpholin-2-yl)éthyl]-4-phénylpipéridin-4-yl}-1,1-diméthylurée, de formule :

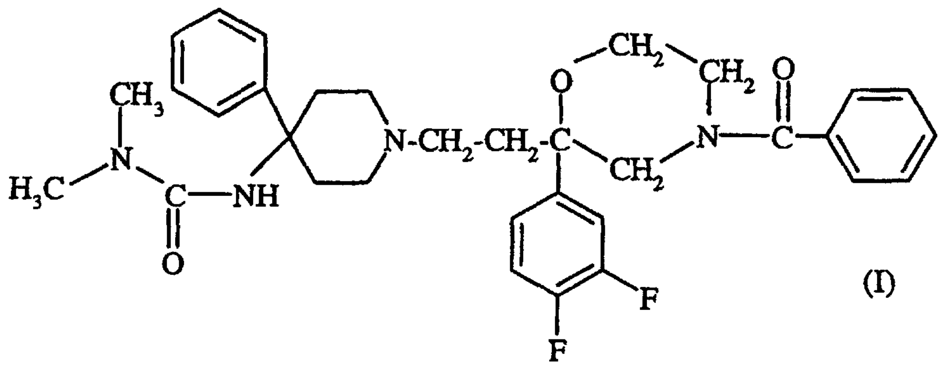

(I)

ci-après dénommé composé A, est un nouvel antagoniste non peptidique puissant et sélectif des récepteurs $NK_2$ de la neurokinine A chez différentes espèces, en particulier des récepteurs $NK_2$ humains (X. Emonds-Alt et al., Neuropeptides, 1997, 31(5), 449-458) et, en conséquence, peut être utile notamment dans le traitement des affections du système respiratoire, gastro-intestinal, urinaire, immunitaire, cardiovasculaire et du système nerveux central ainsi que de la douleur et de la migraine.

La préparation du composé A est illustrée dans la demande internationale WO 96/23787. Selon ce document, le composé A est préparé par réaction du (+)-4-benzoyl-2-(3,4-difluorophényl)-2-[2-(méthanesulfonyloxy)éthyl]morpholine (composé B) avec le para-toluènesulfonate de 3-(4-phénylpipéridin-4-yl)-1,1-diméthylurée (composé C), en présence de carbonate de potassium, puis transformation en son chlorhydrate.

Toutefois ce procédé comporte des désavantages et inconvénients, suffisants pour l'écarter de toute utilisation à l'échelle industrielle.

Par exemple, le composé A préparé par ce procédé est obtenu avec un rendement peu élevé, de l'ordre de 38 % calculé à partir du composé B, d'après la description de la demande WO 96/23787.

La principale raison de ce faible rendement est la formation, lors de la réaction du composé B avec le composé C, de nombreuses impuretés dans le milieu réactionnel, conduisant à un faible taux de transformation en composé A. On a pu isoler et identifier ces impuretés, dont la principale (composé D) a pour formule :

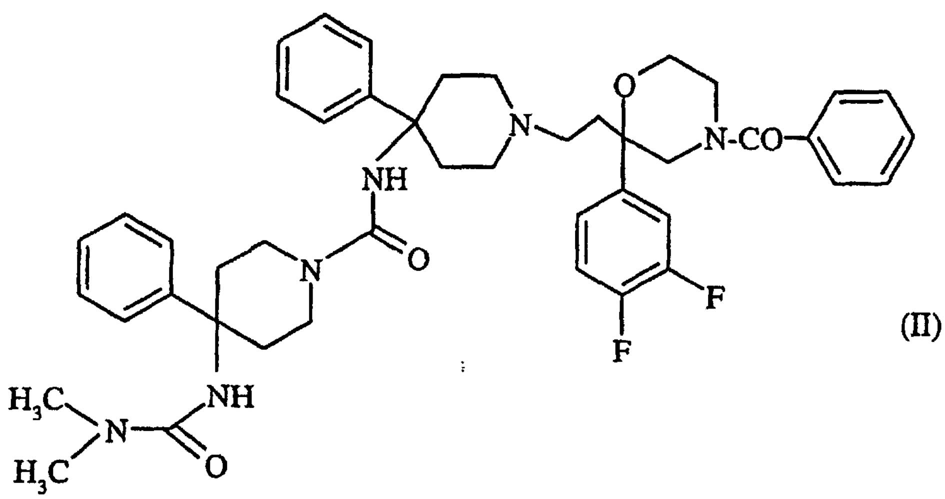

(II)

On a constaté que la formation de ces impuretés, notamment du composé D, est due à l'instabilité du composé C sous forme de base libre en solution. Ainsi une étude de stabilité réalisée sur le composé C à 50°C dans l'acétonitrile

montre qu'il se décompose rapidement (environ 5 % par heure) et donne une multitude de produits correspondant à une polymérisation (dimère, trimère, etc,...), excluant donc l'utilisation du composé C à l'échelle industrielle.

De plus, la présence de ces impuretés, notamment du composé D, dans le milieu réactionnel, rend difficile la séparation et la purification du composé A.

En conséquence, la recherche d'un procédé pour la préparation du composé A ne présentant pas les inconvénients et désavantages du procédé connu de l'art antérieur reste d'un intérêt incontestable.

On a maintenant trouvé un nouveau procédé de préparation du composé A qui met en oeuvre des produits de départ et des composés intermédiaires stables dans les conditions opératoires, et ne conduisant pas à la formation des impuretés présentes lors du procédé de l'art antérieur.

Ainsi, selon un de ses aspects la présente invention a pour objet un procédé de préparation du (R)-(+)-3-{1-[2-(4-benzoyl-2-(3,4-difluorophényl)morpholin-2-yl)éthyl]-4-phénylpipéridin-4-yl}-1,1-diméthylurée, de ses sels, solvats et/ou hydrates, de formule :

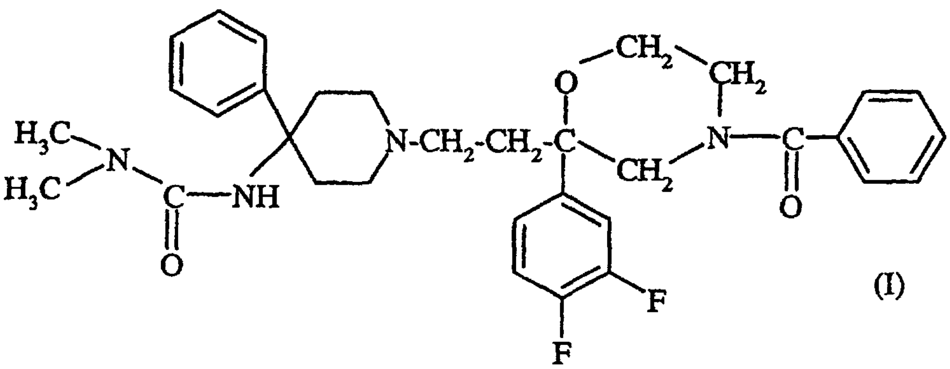

(I)

caractérisé en ce que :

on fait réagir le (+)-4-benzoyl-2-(3,4-difluorophényl)-2-(2-benzènesulfonyloxy éthyl)morpholine de formule :

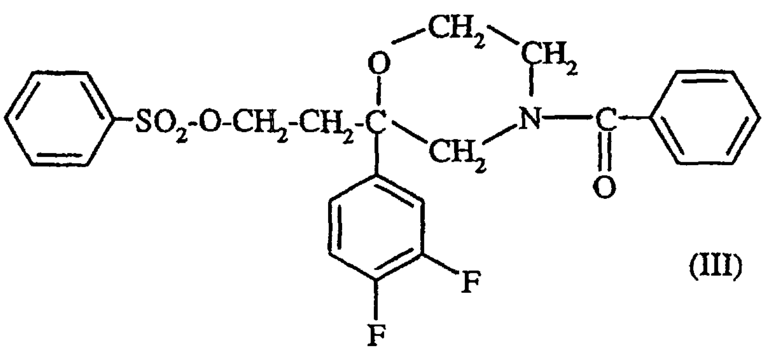

(III)

en présence d'une base, avec l'ester tert-butylique de l'acide (4-phénylpipéridin-4-yl)carbamique de formule :

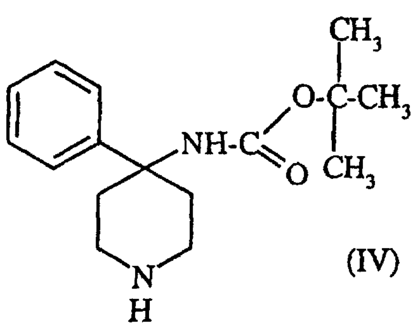

(IV)

pour obtenir l'ester tert-butylique de l'acide (+)-(1-{2-[4-benzoyl-2-(3,4-difluorophényl)morpholin-2-yl]éthyl}-4-phénylpipéridin-4-yl)carbamique, de formule :

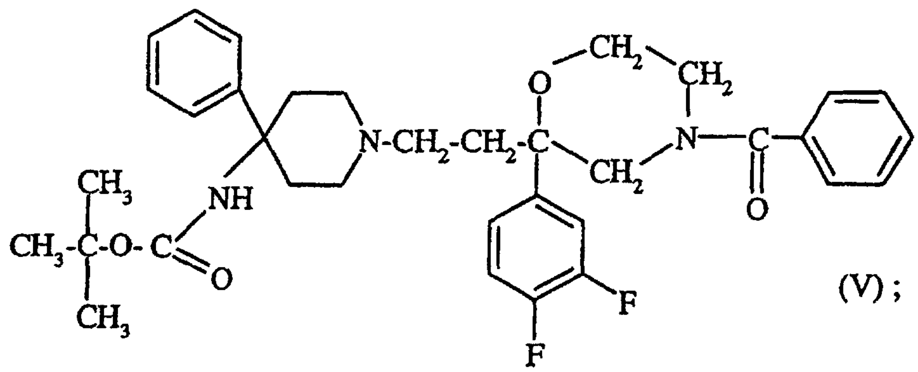

(V) ;

on déprotège le composé de formule (V) ainsi obtenu par action d'un acide, pour obtenir la (+)-[2-[2-(4-amino-4-phénylpipéridin-1-yl)éthyl]-2-(3,4-difluorophényl) morpholin-4-yl]phénylméthanone, de formule :

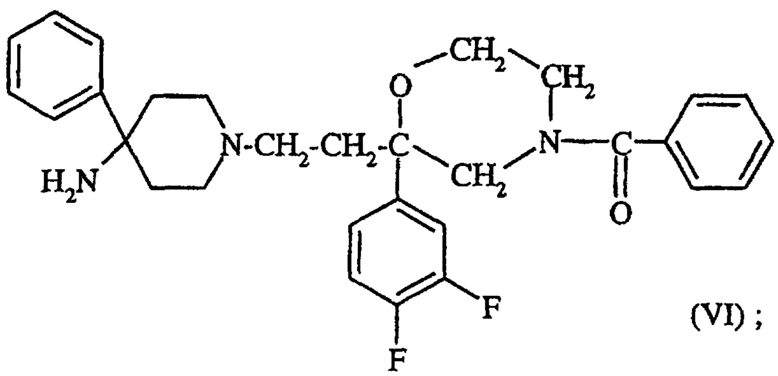

(VI) ;

c) on fait réagir le composé de formule (VI) ainsi obtenu, d'abord avec un dérivé réactif de l'acide carbonique, en présence ou en l'absence de base, puis avec la diméthylamine pour obtenir le composé de formule (I) attendu ;
d) éventuellement, on transforme le composé de formule (I) ainsi obtenu en l'un de ses sels avec des acides minéraux ou organiques pharmaceutiquement acceptables.

Dans le procédé selon l'invention, il est possible de combiner deux ou plusieurs étapes.

Ainsi, par exemple, les étapes a) et b) peuvent être combinées afin d'obtenir directement le composé (VI) à partir du composé de formule (III). De même, les étapes c) et d) peuvent être combinées. Il est aussi possible de combiner toutes les étapes du procédé selon l'invention, ce qui signifie que toutes les étapes sont effectuées sans isoler les composés intermédiaires de formule (V) et (VI), ce qui simplifie le procédé.

On peut former des sels des composés de formule (V) ou (VI) ainsi que du composé de formule (I). Ces sels comprennent aussi bien ceux avec des acides minéraux ou organiques qui permettent une séparation ou une cristallisation convenable des composés de formule (V), (VI) ou (I), que ceux qui forment des sels pharmaceutiquement acceptables avec le composé de formule (I), tels que le chlorhydrate, le bromhydrate, le sulfate, l'hydrogénosulfate, le dihydrogénophosphate, le méthanesulfonate, le méthylsulfate, le maléate, le fumarate, le succinate, le naphtalène-2-sulfonate, le glyconate, le gluconate, le citrate, l'iséthionate, le benzènesulfonate, le paratoluènesulfonate.

Le composé A ainsi obtenu, peut être ultérieurement séparé du milieu réactionnel, selon les méthodes classiques.

Le composé A obtenu est isolé sous forme de base libre ou d'un de ses sels, par exemple le chlorhydrate, le fumarate ou le succinate. Il est aussi possible d'isoler par exemple le composé A sous forme de fumarate et de le convertir en un autre de ses sels, d'abord en le neutralisant puis en traitant la base libre avec un acide, par exemple l'acide succinique.

Lorsque le composé A est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le méthanol, l'éthanol ou le propan-2-ol, ou dans l'acétone, ou dans le dichlorométhane ou dans l'acétate d'éthyle avec une solution de l'acide choisi dans un des solvants précités, on obtient

le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, le méthylsulfate, le benzènesulfonate, le *para*-toluènesulfonate, l'oxalate, le maléate, le succinate, le fumarate, le naphtalène-2-sulfonate, le glyconate, le gluconate, le citrate, l'iséthionate.

De préférence, on utilise le procédé selon l'invention pour préparer le composé A sous forme de succinate ou de fumarate.

Ainsi, le composé A, sous forme de base libre, dissout dans l'acétone est traité, à température ambiante par l'acide succinique dissout dans l'acétone et on obtient le succinate correspondant qui est isolé selon les techniques classiques.

De même, le composé A, sous forme de base libre, dissout dans l'acétone est traité, à chaud, par l'acide fumarique dans l'acétone et on obtient après refrodissement à température ambiante le fumarate correspondant qui est isolé selon les techniques classiques.

Lorsque le composé A est obtenu sous forme d'un de ses sels, par exemple le chlorhydrate ou le fumarate, la base libre peut être préparée par neutralisation dudit sel avec une base minérale ou organique, telle que l'hydroxyde de sodium ou la triéthylamine, ou avec un carbonate ou bicarbonate de métal alcalin, tel que le carbonate ou bicarbonate de sodium ou de potassium selon les méthodes classiques.

Lors de la mise en oeuvre du procédé selon l'invention, on a pu obtenir le composé A ou l'un de ses sels avec un rendement final de l'ordre de 55 à 70 % calculés par rapport au composé de formule (III) de départ.

La présente invention a donc pour objet un procédé de préparation du (R)-(+)-3-{1-[2-(4-benzoyl-2-(3,4-difluorophényl)morpholin-2-yl)éthyl]-4-phénylpipéridin-4-yl}-1,1-diméthylurée, de ses sels, solvats et/ou hydrates, de formule :

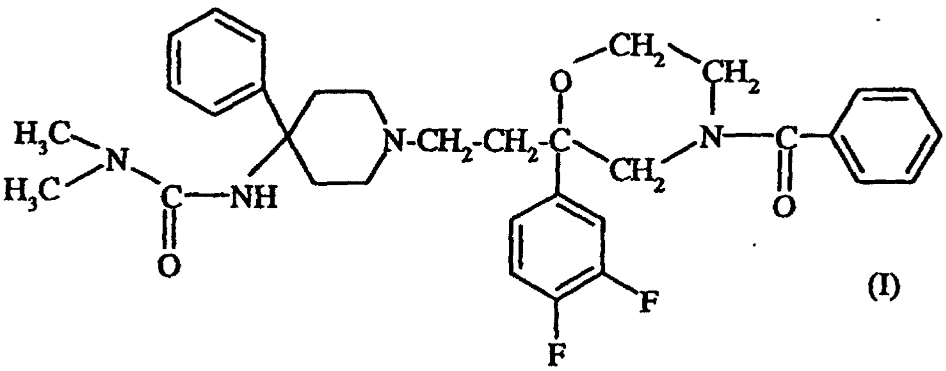

(I)

caractérisé en ce que l'on fait réagir la (+)-[2-[2-(4-amino-4-phénylpipéridin-1-yl)éthyl]-2-(3,4-difluorophényl)morpholin-4-yl]phénylméthanone, de formule :

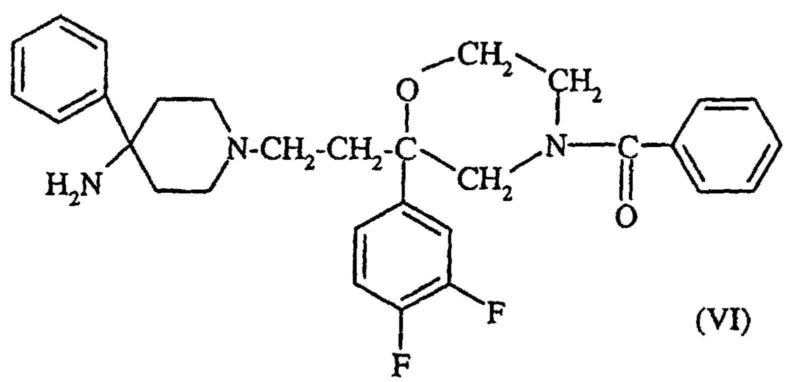

(VI)

dans un solvant inerte, d'abord avec un dérivé réactif de l'acide carbonique en présence ou en l'absence de base, puis avec la diméthylamine, et, éventuellement on transforme le composé de formule (I) ainsi obtenu en l'un des sels avec des acides minéraux ou organiques pharmaceutiquement acceptables.

Parmi les dérivés réactifs de l'acide carbonique, on préfère le 1,1'-carbonyldiimidazole, le phosgène ou le chloroformiate de p-nitrophényle.

Particulièrement, on préfère, selon l'invention, utiliser le 1,1'-carbonyldiimidazole.

Le dérivé réactif de l'acide carbonique intervient dans la réaction à raison de 1 à 3 équivalents molaires par équivalent molaire de composé de formule (VI), de préférence de 1 à 2 équivalents molaires.

Lorsqu'on utilise le 1,1'-carbonyldiimidazole, la réaction s'effectue en l'absence de base. Lorsqu'on utilise le phosgène ou le chloroformiate de *p*-nitrophényle, la réaction s'effectue en présence d'une base organique telle que la triéthylamine, la N,N-diisopropyléthylamine ou la N-méthylmorpholine ; de préférence on utilise la triéthylamine.

La base intervient dans la réaction à raison de 1 à 6 équivalents molaires par équivalent molaire de dérivés réactifs de l'acide carbonique.

La diméthylamine intervient dans la réaction à raison de 1 à 6 équivalents molaires par équivalent molaire de composé de formule (VI), de préférence de 1 à 4 équivalents molaires.

Le solvant inerte peut être par exemple un hydrocarbure aliphatique halogéné en $C_1$-$C_4$ tel que le dichlorométhane, le 1,2-dichloroéthane, le chloroforme, le tétrachlorure de carbone. Le dichlorométhane constitue un solvant préféré.

Le solvant inerte est utilisé à raison de 2 à 15 équivalents en volume par équivalent en poids de composé de formule (VI). De préférence, on utilise le solvant à raison de 5 à 10 équivalents en volume par équivalent en poids de composé de formule (VI).

La réaction est conduite à une température comprise entre -20°C et 25°C.

La réaction ainsi décrite se déroule sur une période de 4 à 15 heures.

Le composé de formule (VI) et ses sels sont nouveaux et font partie de l'invention.

Selon un autre de ses aspects, l'invention a pour objet un procédé de préparation du (+)-[2-[2-(4-amino-4-phénylpipéridin-1-yl)éthyl]-2-(3,4-difluorophényl)morpholin-4-yl]phénylméthanone, ou de l'un de ses sels, de formule :

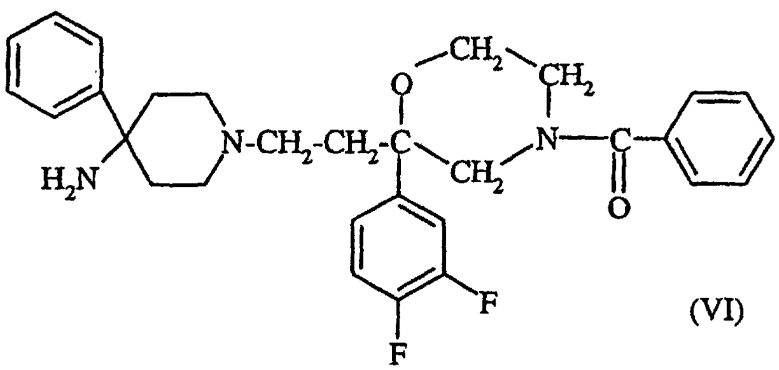

(VI)

caractérisé en ce que l'on déprotège l'ester tert-butylique de l'acide (+)-(1-{2-[4-benzoyl-2-(3,4-difluorophényl)morpholin-2-yl]éthyl}-4-phénylpipéridin-4-yl) carbamique, de formule :

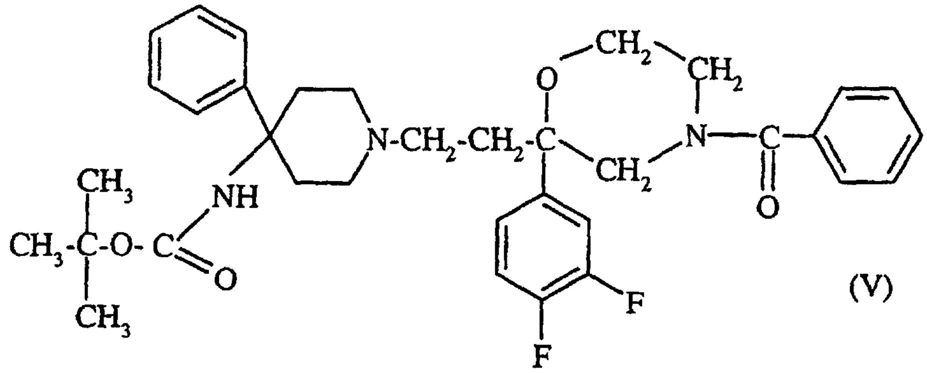

(V)

par action d'un acide, dans un solvant inerte, et, éventuellement, on transforme le composé de formule (VI) ainsi obtenu en un de ses sels.

Pour effectuer la déprotection on utilise de préférence un acide fort tel que l'acide chlorhydrique, l'acide trifluoroacétique ou l'acide formique. L'acide chlorhydrique peut être généré *in-situ* à partir de chlorure d'acétyle et de

méthanol.

Particulièrement, on préfère utiliser l'acide chlorhydrique.

L'acide intervient dans la réaction à raison de 4 à 10 équivalents molaires par équivalent molaire de composé de formule (V), de préférence de 4 à 6 équivalents molaires.

Le solvant inerte peut être par exemple la méthylisobutylcétone, le dichlorométhane, l'acétate d'éthyle, le toluène ou un mélange de ces solvants. La méthylisobutylcétone constitue un solvant préféré.

Le solvant inerte est utilisé à raison de 2 à 15 équivalents en volume par équivalent en poids de composé de formule (V). De préférence, on utilise le solvant à raison de 2 à 5 équivalents en volume par équivalent en poids de composé de formule (V).

La réaction est conduite à une température comprise entre 10°C et 60°C, de préférence à une température comprise entre 20°C et 40°C.

La réaction se déroule sur une période de 30 minutes à 18 heures.

Le composé (VI) ainsi obtenu peut être ultérieurement séparé du milieu réactionnel, selon les méthodes classiques.

Le composé de formule (VI) obtenu est isolé sous forme de base libre ou d'un de ses sels, en utilisant les méthodes précédemment citées pour le composé A.

Le composé de formule (V) et ses sels sont nouveaux et font partie de l'invention.

Selon un autre de ses aspects, l'invention a pour objet un procédé de préparation de l'ester *tert*-butylique de l'acide (+)-(1-{2-[4-benzoyl-2-(3,4-difluorophényl) morpholin-2-yl]éthyl}-4-phénylpipéridin-4-yl) carbamique, ou de l'un de ses sels, de formule :

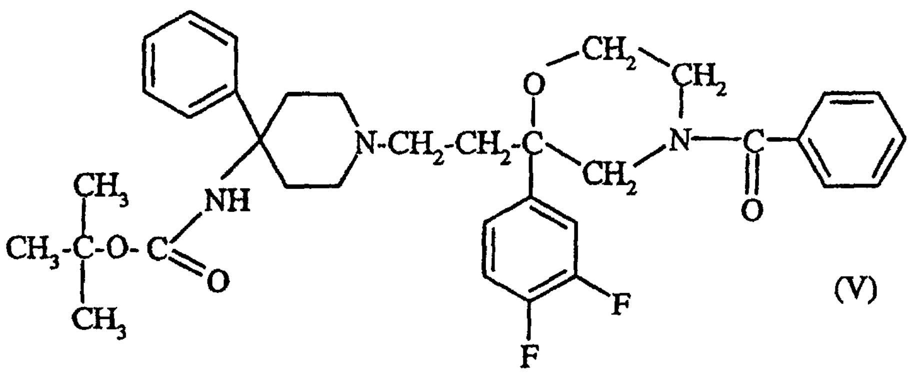

(V)

caractérisé en ce que l'on fait réagir le (+)-4-benzoyl-2-(3,4-difluorophényl)-2-(2-benzènesulfonyloxyéthyl)morpholine de formule :

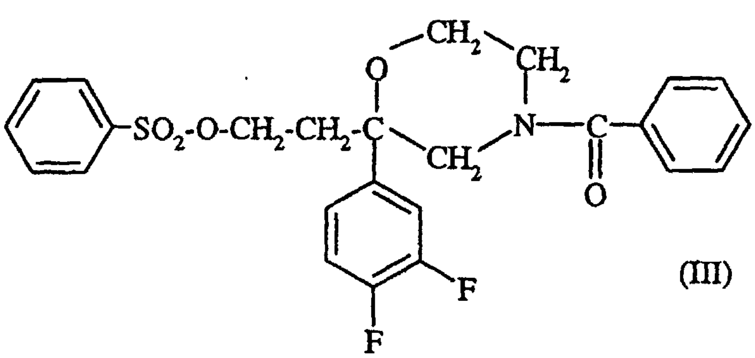

(III)

avec l'ester *tert*-butylique de l'acide (4-phénylpipéridin-4-yl)carbamique, de formule :

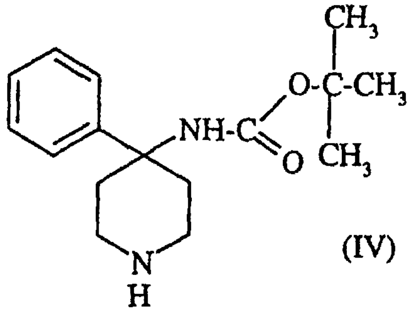

(IV)

en présence d'une base, dans un solvant inerte en mélange avec de l'eau, et éventuellement, on transforme le composé de formule (V) ainsi obtenu en un de ses sels.

Le composé de formule (IV) intervient dans la réaction à raison de 1 à 1,25 équivalents molaires par équivalent molaire de composé de formule (III).

La base utilisée lors de la réaction est choisie parmi un hydroxyde de métal alcalin tels que l'hydroxyde de sodium ou l'hydroxyde de potassium, ou parmi les carbonates ou bicarbonates de métal alcalin tels que le carbonate de potassium, le carbonate de sodium ou le bicarbonate de sodium. De préférence, on utilise le carbonate de potassium.

La base intervient dans la réaction à raison de 1 à 3 équivalents molaires par équivalent molaire de composé de formule (III).

L'eau intervient dans la réaction à raison de 1 à 3 équivalents en volume par équivalent en poids de base.

Le solvant inerte peut être par exemple la méthylisobutylcétone, le toluène, l'acétonitrile, l'éthanol, ou un mélange de ces solvants. La méthylisobutylcétone constitue un solvant préféré.

Le solvant inerte est utilisé à raison de 2 à 15 équivalents en volume par équivalent en poids de composé de formule (III). De préférence, on utilise le solvant à raison de 2 à 5 équivalents en volume par équivalent en poids de composé de formule (III).

La réaction est conduite à une température comprise entre 20°C et 90°C.

La réaction se déroule sur une période de 2 à 30 heures.

Le composé de formule (V) ainsi obtenu, peut être ultérieurement séparé du milieu réactionnel selon les méthodes classiques.

Le composé de formule (V) obtenu est isolé sous forme de base libre ou d'un de ses sels, en utilisant les méthodes précédemment citées pour le composé A.

De manière alternative, on peut aussi préparer, dans le procédé selon l'invention, le composé de formule (VI) directement et en une seule étape à partir du composé de formule (III), ce qui simplifie le procédé.

Selon un autre de ses aspects, l'invention a pour objet un autre procédé de préparation du (+)-[2-[2-(4-amino-4-phénylpipéridin-1-yl)éthyl]-2-(3,4-difluorophényl) morpholin-4-yl]phénylméthanone, ou l'un de ses sels, de formule :

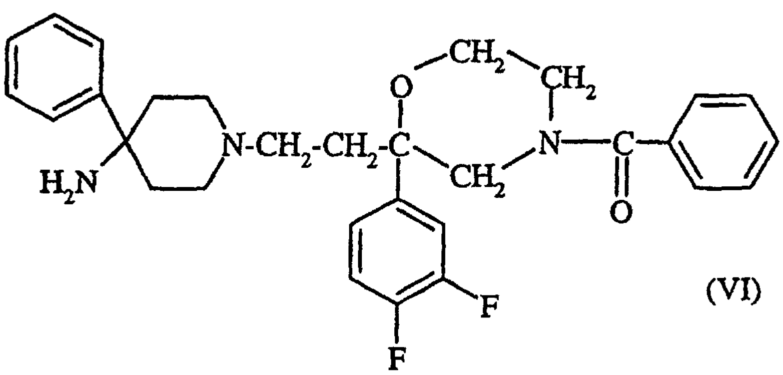

(VI)

caractérisé en ce que l'on fait réagir le (+)-4-benzoyl-2-(3,4-difluorophényl)-2-(2-benzènesulfonyloxyéthyl)morpholine de formule :

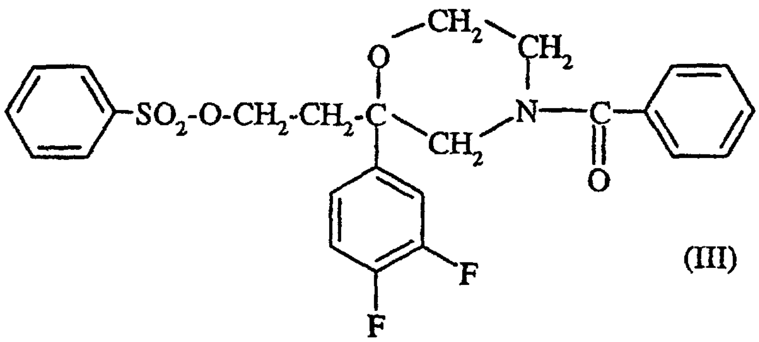

(III)

avec le 4-amino-4-phénylpipéridine de formule :

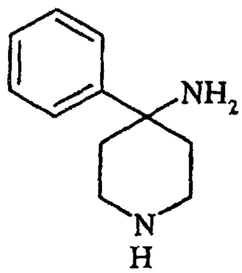

(IX)

en présence d'une base, dans un solvant inerte en mélange avec de l'eau, et éventuellement, on transforme le composé de formule (VI) ainsi obtenu en un de ses sels.

Le composé de formule (IX) intervient dans la réaction à raison de 1 à 1,25 équivalents molaires par équivalent molaire de composé de formule (III).

La base utilisée lors de la réaction est choisie parmi un hydroxyde de métal alcalin tels que l'hydroxyde de sodium ou l'hydroxyde de potassium, ou parmi les carbonates ou bicarbonates de métal alcalin tels que le carbonate de potassium, le carbonate de sodium ou le bicarbonate de sodium. De préférence, on utilise le carbonate de potassium.

La base intervient dans la réaction à raison de 1 à 3 équivalents molaires par équivalent molaire de composé de formule (III).

L'eau intervient dans la réaction à raison de 1 à 3 équivalents en volume par équivalent en poids de base.

Le solvant inerte peut être par exemple la méthylisobutylcétone, le toluène, l'acétonitrile, l'éthanol, ou un mélange de ces solvants. La méthylisobutylcétone constitue un solvant préféré.

Le solvant inerte est utilisé à raison de 2 à 15 équivalents en volume par équivalent en poids de composé de formule (III). De préférence, on utilise le solvant à raison de 2 à 5 équivalents en volume par équivalent en poids de composé de formule (III).

La réaction est conduite à une température comprise entre 20°C et 90°C.

La réaction se déroule sur une période de 2 à 30 heures.

Le composé de formule (VI) ainsi obtenu, peut être ultérieurement séparé du milieu réactionnel selon les méthodes classiques.

Le composé de formule (VI) obtenu est isolé sous forme de base libre ou d'un de ses sels, en utilisant les méthodes précédemment citées pour le composé A.

Le composé de formule (IV) est connu et se prépare selon des méthodes connues telles que celles décrites dans EP-A-0 673 928.

Le composé de formule (IX) est connu et se prépare selon des méthodes connues telles que celles décrites dans WO 97/10211, en particulier, par déprotection selon les méthodes classiques de la 4-amino-1-benzyl-4-phénylpipéridine connue et préparée selon WO 97/10211 ou WO 96/23787.

Le composé de formule (III) est nouveau et fait partie de l'invention.

Le composé de formule (III) se prépare selon des méthodes connues telles que celles décrites dans WO 96/23787.

En particulier, le composé de formule (III) se prépare selon le SCHEMA ci-après.

SCHEMA 1

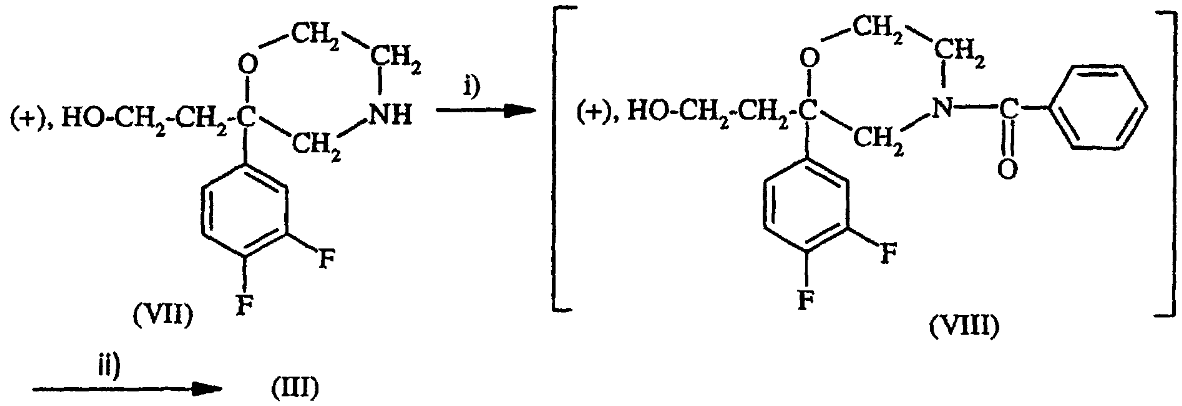

(VII)
(VIII)
(III)

A l'étape i), le composé de formule (VII) est mis en réaction avec le chlorure de benzoyle, en présence d'une base telle que l'hydroxyde de sodium, dans un solvant inerte tel que le dichlorométhane ou le toluène en mélange avec de l'eau et à une température comprise entre 10°C et 35°C. Après extraction, et lavage à l'eau, le composé de formule (VIII) en solution dans la phase organique est mis en réaction (étape ii)) avec le chlorure de benzènesulfonyle, en présence d'un catalyseur de transfert de phase tel que le chlorure de benzyltriéthylammonium, d'une base telle que l'hydroxyde de sodium et d'eau et à une température comprise entre la température ambiante et 55°C. Après hydrolyse à l'eau, le composé de formule (III) est isolé selon les méthodes classiques.

Le composé de formule (III) peut être mis en réaction sans être isolé du milieu dans lequel il a été produit. Le composé de formule (VII) est connu et se prépare selon des méthodes connues, telles que celles décrites dans WO 96/23787 ou précisément dans Tetrahedron : Asymmetry, 1998 9, 3251-3262.

Les Exemples non limitatifs suivants illustrent l'invention.

EXEMPLE 1

(+)-4-Benzoyl-2-(3,4-difluorophényl)-2-(2-benzènesulfonyloxyéthyl)morpholine, composé de formule (III).

A un mélange de 17,74 g de (+)-2-[2-(3,4-difluorophényl)morpholin-2-yl]éthanol (composé VII) dans 180 ml de dichlorométhane, on ajoute 160 ml d'eau, puis 18,2 ml d'une solution à 30 % d'hydroxyde de sodium dans l'eau. On met le mélange sous forte agitation et ajoute rapidement, en 3 minutes, 10,25 g de chlorure de benzoyle, la température s'élevant de 20,7°C à 30,7°C. On laisse sous agitation en laissant la température revenir à 27°C. Après décantation, on extrait la phase aqueuse par 5 ml de dichlorométhane et joint les phases organiques contenant le composé (VIII).

On met les phases organiques jointes sous agitation à 350 tours/minute, ajoute 0,85 g de chlorure de benzyltriéthylammonium, puis une solution de 27 g d'hydroxyde de sodium dans 27 ml d'eau, la température s'élevant à 30°C. On ajoute alors rapidement 25,8 g de chlorure de benzènesulfonyle, la température s'élevant lentement jusqu'à 34,7°C, et laisse 18 heures sous agitation en maintenant la température à 32°C. On hydrolyse le mélange réactionnel par ajout de 200 ml d'eau, puis, après décantation, concentre sous vide la phase organique. On reprend le résidu dans 200 ml de toluène, lave successivement la phase organique par 100 ml d'eau, deux fois par 100 ml de tampon sulfate pH = 2, par 100 ml d'eau, par 100 ml d'une solution saturée de chlorure de sodium, sèche sur sulfate de magnésium et, après filtration, évapore sous vide le solvant. On reprend l'huile obtenue dans 100 ml d'éther diisopropylique, évapore à nouveau sous vide le solvant et répète en tout trois fois cette opération. On reprend le produit obtenu dans 100 ml d'un mélange éther diisopropylique/éther diéthylique (50/50 ; v/v), refroidit à 0°C et laisse en concrétisation pendant 2 heures. On abandonne une nuit à température ambiante, refroidit à 0°C pendant 1 heure, essore le précipité formé et le sèche sous vide. On obtient 31,6 g du produit attendu.
Rendement : 89 %.
Point de fusion : 82,8°C
$\alpha_D^{20}$ = +46,9° (c = 1 ; MeOH)

EXEMPLE 2

Ester *tert*-butylique de l'acide (+)-(1-{2-[4-benzoyl-2-(3,4-difluorophényl)morpholin-2-yl]éthyl}-4-phénylpipéridin-4-yl) carbamique, composé de formule (V).

A un mélange de 22,6 g de chlorhydrate de l'ester tert-butylique de l'acide (4-phénylpipéridin-4-yl)carbamique (composé (IV)) et de 30 g de composé de formule (III) obtenu à l'EXEMPLE 1 dans 300 ml d'acétonitrile, on ajoute une solution de 18 g de carbonate de potassium dans 18 ml d'eau, puis chauffe à 70°C pendant 12 heures et abandonne une nuit à température ambiante. On concentre sous vide l'acétonitrile, reprend le résidu dans 300 ml de dichlorométhane, lave successivement la phase organique par 150 ml d'une solution 0,3 M d'hydroxyde de sodium dans l'eau, par 150 ml d'eau, deux fois par 150 ml de tampon sulfate pH = 2, par 150 ml d'eau, par 150 ml d'une solution à 10 % en poids de carbonate de potassium dans l'eau, sèche sur sulfate de magnésium, filtre et évapore sous vide le solvant à température ambiante. On obtient le produit attendu qui est mis en ouvre à l'EXEMPLE 3.
Point de fusion : 106,8°C
$\alpha_D^{20}$ = +16,2° (c = 1 ; MeOH)

EXEMPLE 3

Dichlorhydrate de (+)-[2-[2-(4-amino-4-phénylpipéridin-1-yl)éthyl]-2-(3,4-difluorophényl)morpholin-4-yl]phénylméthanone, composé de formule (VI).

On refroidit à -10°C 250 ml de méthanol, ajoute rapidement 25 ml de chlorure d'acétyle et laisse revenir à température ambiante. On ajoute alors le composé de formule (V) obtenu à l'EXEMPLE 2 et laisse une nuit sous agitation à température ambiante. On concentre sous vide le solvant à température ambiante, reprend le résidu par 100 ml de méthanol et concentre sous vide à nouveau le solvant. On reprend le résidu par 100 ml d'acétate d'éthyle, concentre sous vide le solvant et répète en tout trois fois cette opération. On reprend le résidu par 150 ml d'acétate d'éthyle, laisse deux heures sous agitation à température ambiante, essore le précipité formé, le lave par de l'acétate d'éthyle et le sèche sous vide. On obtient 32,4 g du produit attendu.
Rendement : 91 % (calculé à partir du composé (III))
Point de fusion : 272,2°C
$\alpha_D^{20}$ (base libre) = +17,6° (c = 1 ; MeOH)

EXEMPLE 3 bis

(+)-[2-[2-(4-amino-4-phénylpipéridin-1-yl)éthyl]-2-(3,4-difluorophényl)morpholin-4-yl]phénylméthanone, composé de formule (VI).

4-Amino-4-phénylpipéridine.

A une solution de 5 g de 4-amino-1-ben2yl-4-phénylpipéridine dans 25 ml de méthanol, on ajoute 3,2 ml d'une solution 12N d'acide chlorhydrique puis 0,5 g de palladium sur charbon à 10 % (50 % d'humidité) et hydrogène pendant une nuit, à pression atmosphérique et à 40°C. On filtre le catalyseur et concentre sous vide le filtrat. On reprend le résidu dans 15 ml d'eau, alcanilise par ajout de 3,8 ml d'une solution 10N d'hydroxyde de sodium et laisse sous agitation. On ajoute du chlorure de sodium en cristaux dans la phase aqueuse et extrait deux fois par 25 ml de dichlorométhane. On sèche la phase organique sur sulfate de sodium, filtre et évapore sous vide le solvant. On obtient 3,4 g du produit attendu.
b) A une solution de 4 g de composé de formule (III) obtenu à l'EXEMPLE 1 dans 9,2 ml de toluène et 8 ml de méthylisobutylcétone, on ajoute une solution de 2,83 g de carbonate de potassium dans 2,8 ml d'eau, puis 1,6 g de 4-amino-4-phénylpipéridine et chauffe à 70°C pendant 12 heures. Après refrodissement du mélange réactionnel à température ambiante, on ajoute 16 ml d'eau puis décante. On lave deux fois la phase organique par 16 ml d'eau, acidifie la phase organique par ajout d'une solution de 1,5 ml d'acide chlorhydrique 12N dans 16 ml d'eau puis décante. On lave deux fois la phase aqueuse acide par 10 ml de toluène, alcalinise la phase aqueuse par ajout de 2 ml d'une solution 10 N d'hydroxyde de sodium et extrait deux fois par 30 ml de toluène. On sèche la phase organique sur sulfate de magnésium, filtre et évapore sous vide le solvant. On obtient 4 g du produit attendu sous forme d'huile.

EXEMPLE 4

Fumarate de (R)-(+)-3-{1-[2-(4-benzoyl-2-(3,4-difluorophényl)morpholin-2-yl)éthyl]-4-phénylpipéridin-4-yl}-1,1-diméthylurée, composé A.

A une suspension de 32,3 g du composé de formule (VI) obtenu à l'EXEMPLE 3 dans 150 ml de dichlorométhane, on ajoute 100 ml d'une solution à 20 % en poids de carbonate de potassium et agite vigoureusement. Après décantation, on extrait la phase aqueuse par 50 ml de dichlorométhane, joint les phases organiques, les sèche sur sulfate de magnésium, filtre et ajoute à cette solution 16,5 ml de triéthylamine. On coule ensuite cette solution, sous atmosphère d'azote, en deux heures 15 minutes et en maintenant la température du milieu réactionnel entre -8°C et -10°C, sur 42 ml d'une solution 1,4M de phosgène dans le toluène préalablement refroidie à -10°C, puis laisse 30 minutes sous agitation à -10°C. On ajoute rapidement 5 ml d'une solution 1,4M de phosgène dans le toluène, laisse 5 minutes sous agitation et, à nouveau ajoute 1 ml d'une solution 1,4M de phosgène dans le toluène. On coule alors, en 3 minutes, à -12°C, 50 ml d'une solution 2M de diméthylamine dans le tétrahydrofurane, puis laisse sous agitation en laissant remonter la température à température ambiante. On verse le mélange réactionnel dans 200 ml de tampon sulfate pH = 2, après décantation, lave successivement la phase organique par 200 ml de tampon sulfate pH = 2, par 200 ml d'eau, par 200 ml d'une solution à 10 % de carbonate de potassium dans l'eau, sèche sur sulfate de magnésium, filtre et concentre sous vide le filtrat. On dissout le résidu dans 50 ml d'acétone et coule cette solution sur un mélange de 6,4 g d'acide fumarique dans 256 ml d'acétone, préalablement chauffé à reflux. Après l'addition, on maintient le milieu sous agitation à 57°C pendant 15 minutes, puis laisse revenir à 26°C. On essore le précipité formé, le lave deux fois par 50 ml d'acétone et le sèche sous vide une nuit à température ambiante. On obtient ainsi 29,1 g du composé A sous forme de fumarate.
Rendement : 75 % (calculé à partir du composé VI)
68,25 % (calculé à partir du composé III).
Point de fusion : 201,5°C
$\alpha_D^{20}$ = +19,1° (c = 1 ; MeOH).

EXEMPLE 5

Succinate de (R)-(+)-3-{1-[2-(4-benzoyl-2-(3,4-difluorophényl)morpholin-2-yl)éthyl]-4-phénylpipéridin-4-yl}-1,1-diméthylurée, composé A.

a) A un mélange de 92,6 g de composé de formule (III) dans 200 ml de méthylisobutylcétone, on ajoute sous agitation 58 g d'ester tert-butylique de l'acide (4-phénylpipéridin-4-yl)carbamique (composé (IV)) puis coule une solution de 31,6 g de carbonate de potassium dans 32 ml d'eau et chauffe à 70°C pendant 23 heures en maintenant sous agitation. On laisse revenir le mélange réactionnel à 50°C, ajoute, en 1 heure, 460 ml d'eau, refroidit à 30°C et décante. La phase organique contenant le composé de formule (V) est engagée directement dans l'étape suivante.
b) On chauffe à 30°C et sous agitation la solution de composé de formule (V) dans la méthylisobutylcétone obtenue à l'étape a), puis coule, en 45 minutes et en goutte à goutte, 80 ml d'une solution 12M d'acide chlorhydrique dans l'eau. A la fin de l'addition, on ajoute 380 ml d'eau au mélange réactionnel, décante, élimine la phase organique, lave la phase aqueuse par 380 ml d'acétate d'éthyle, alcalinise la phase aqueuse par ajout de 115 ml d'une solution 10M d'hydroxyde de sodium dans l'eau, extrait par 380 ml de toluène et lave quatre fois la phase organique par 380 ml d'eau. On ajoute 380 ml d'eau à la phase organique, acidifie le mélange par ajout de 32 ml d'une solution 12M d'acide chlorhydrique dans l'eau, élimine la phase organique, alcalinise la phase aqueuse par ajout de 42 ml d'une solution 10M d'hydoxyde de sodium dans l'eau et extrait trois fois par 380 ml de dichlorométhane. On sèche la phase organique sur du sulfate de magnésium, filtre et concentre sous vide le filtrat jusqu'à un poids final de solution de 330 g. La phase organique contenant le composé de formule (VI) est engagée dans l'étape suivante.
c) On refroidit à 0°C une suspension de 60,7 g de 1,1'-carbonyidiimidazole dans 607 ml de dichlorométhane, ajoute sous atmosphère d'azote, sous agitation, en 3 heures et goutte à goutte, la solution de composé de formule (VI) dans le dichlorométhane obtenue à l'étape b) en maintenant la température du milieu réactionnel à 0°C. A la fin de l'addition, on refroidit le mélange réactionnel, à 0°C et ajoute en 45 minutes, sous agitation, par barbottage, 32,8 g de diméthylamine gaz. La température du milieu réactionnel s'élève jusqu'à 10°C, pour être de 3°C à la fin de l'addition. On laisse revenir sous agitation le mélange réactionnel à 20°C, puis ajoute 1000 ml d'eau, laisse 15 minutes sous agitation, élimine la phase aqueuse et répète trois fois cette opération. On sèche la phase organique sur sulfate de magnésium, filtre et évapore sous vide le solvant. On obtient 112,6 g d'une huile contenant une

teneur en poids de 78 % en composé A.
d) A une solution de 65,9 g d'huile ci-dessus (soit 51,4 g en composé A) dans 52 ml d'acétone, on coule, en quelques minutes à température ambiante, une solution de 10,5 g d'acide succinique dans 425 ml d'acétone, puis laisse sous agitation pendant 24 heures. On essore le précipité formé, le lave trois fois par 50 ml d'acétone et le sèche une nuit sous vide à température ambiante. On obtient 48,7 g du composé A sous forme de succinate.

Rendement de la salification : 78,7 %
Rendement global : 63,1 % (calculé à partir du composé III).
Point de fusion : 155°C
$\alpha_D^{20}$ = +17,5° (c = 1 ; MeOH).

**Revendications**

1. Procédé de préparation du (R)-(+)-3-{1-[2-(4-benzoyl-2-(3,4-difluorophényl)morpholin-2-yl)éthyl]-4-phényl pipéridin-4-yl}-1,1-diméthylurée, de ses sels, solvats et/ou hydrates, de formule :

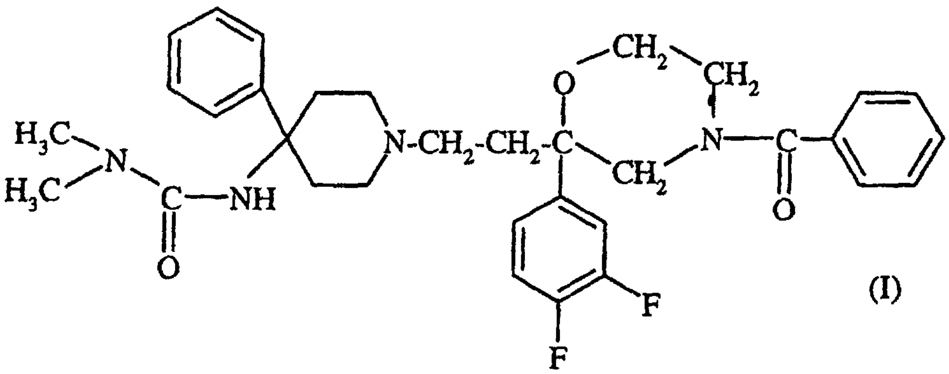

(I)

**caractérisé en ce que** l'on fait réagir la (+)-[2-[2-(4-amino-4-phénylpipéridin-1-yl)éthyl]-2-(3,4-difluorophényl)morpholin-4-yl]phénylméthanone, de formule :

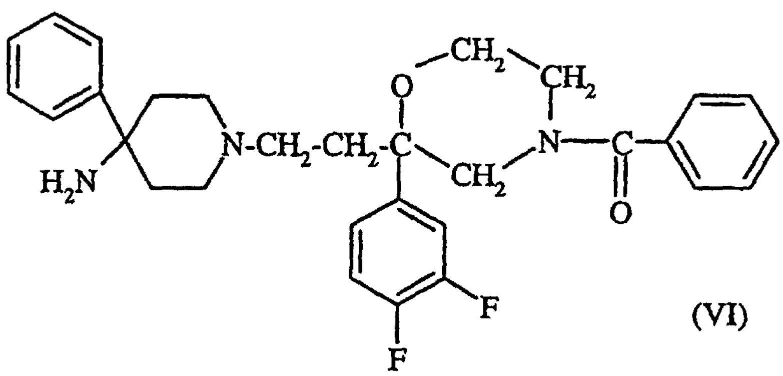

(VI)

dans un solvant inerte, d'abord avec un dérivé réactif de l'acide carbonique en présence ou en l'absence de base, puis avec la diméthylamine, et, éventuellement on transforme le composé de formule (I) ainsi obtenu en l'un des sels avec des acides minéraux ou organiques pharmaceutiquement acceptables.

2. Procédé selon la revendication 1 **caractérisé en ce que** le dérivé réactif de l'acide carbonique est choisi parmi le 1,1'-carbonyldiimidazole, le phosgène ou le chloroformiate de *p*-nitrophényle.

3. Procédé selon l'une des revendications 1 ou 2 **caractérisé en ce que** le dérivé réactif de l'acide carbonique est utilisé à raison de 1 à 3 équivalents molaires par équivalent molaire de composé de formule (VI).

**4.** Procédé selon l'une des revendications 1 à 3 **caractérisé en ce que**, lorsqu'on utilise le phosgène ou le chloroformiate de p-nitrophényle, la réaction s'effectue en présence d'une base choisie parmi la triéthylamine, la N,N-diisopropyléthylamine ou la N-méthylmorpholine.

**5.** Procédé selon l'une des revendications 1 à 4 **caractérisé en ce que** la base est utilisée à raison de 1 à 6 équivalents molaires par équivalent molaire de dérivés réactifs de l'acide carbonique.

**6.** Procédé selon l'une des revendications 1 à 5 **caractérisé en ce que** la diméthylamine est utilisée à raison de 1 à 6 équivalents molaires par équivalent molaire de composé de formule (VI).

**7.** Procédé selon l'une des revendications 1 à 6 **caractérisé en ce que** le solvant inerte est le dichlorométhane, le 1,2-dichloroéthane, le chloroforme ou le tétrachlorure de carbone.

**8.** Procédé selon l'une des revendications 1 à 7 **caractérisé en ce que** la réaction est conduite à une température comprise entre -20°C et 25°C.

**9.** Procédé selon la revendication 1 **caractérisé en ce que** l'on prépare le (+)-[2-[2-(4-amino-4-phénylpipéridin-1-yl) éthyl]-2-(3,4-difluorophényl)morpholin-4-yl]phénylméthanone, ou l'un de ses sels, de formule :

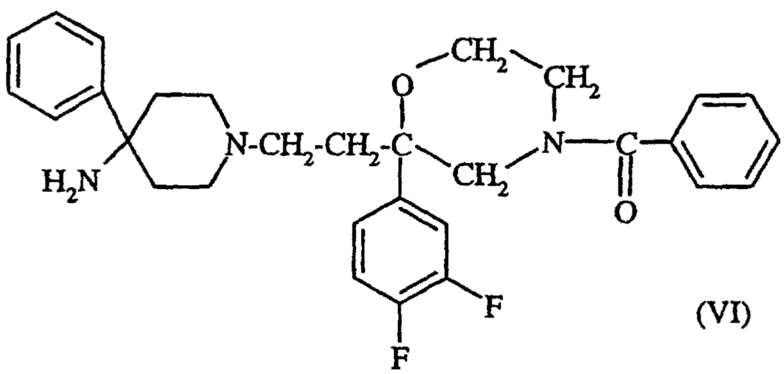

(VI)

par réaction de l'ester *tert*-butylique de l'acide (+)-(1-{2-[4-benzoyl-2-(3,4-difluorophényl)morpholin-2-yl]éthyl}-4-phénylpipéridin-4-yl) carbamique, de formule :

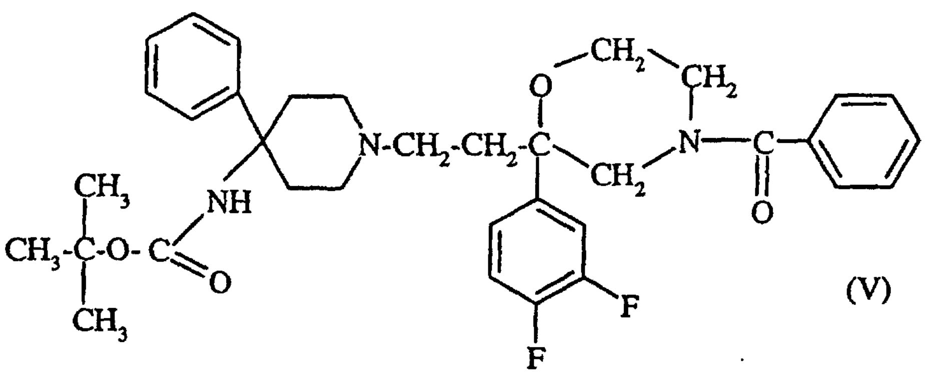

(V)

avec un acide, dans un solvant inerte, et, éventuellement, on transforme le composé de formule (VI) ainsi obtenu en un de ses sels.

**10.** Procédé selon la revendication 9 **caractérisé en ce que** l'acide est l'acide chlorhydrique, l'acide trifluoroacétique ou l'acide formique.

**11.** Procédé selon l'une des revendications 9 ou 10 **caractérisé en ce que** l'acide est utilisé à raison de 4 à 10 équivalents molaires par équivalent molaire de composé de formule (V).

**12.** Procédé selon l'une des revendications 9 à 11 **caractérisé en ce que** le solvant inerte est la méthylisobutylcétone, le dichlorométhane, l'acétate d'éthyle, le toluène ou un mélange de ces solvants.

**13.** Procédé selon l'une des revendications 9 à 12 **caractérisé en ce que** la réaction est conduite à une température comprise entre 10°C et 60°C.

**14.** Procédé selon la revendication 9 **caractérisé en ce que** l'on prépare l'ester tert-butylique de l'acide (+)-(1-{2-[4-benzoyl-2-(3,4-difluorophényl)morpholin-2-yl]éthyl}-4-phénylpipéridin-4-yl) carbamique, ou l'un de ses sels, de formule :

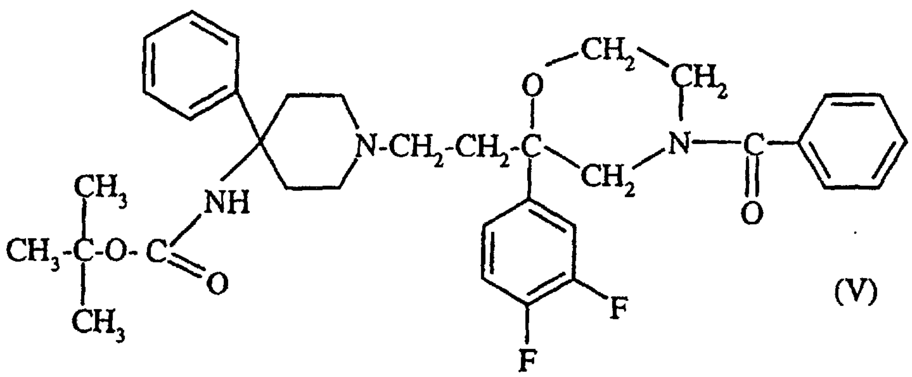

(V)

par réaction du (+)-4-benzoyl-2-(3,4-difluorophényl)-2-(2-benzènesulfonyl oxyéthyl)morpholine de formule :

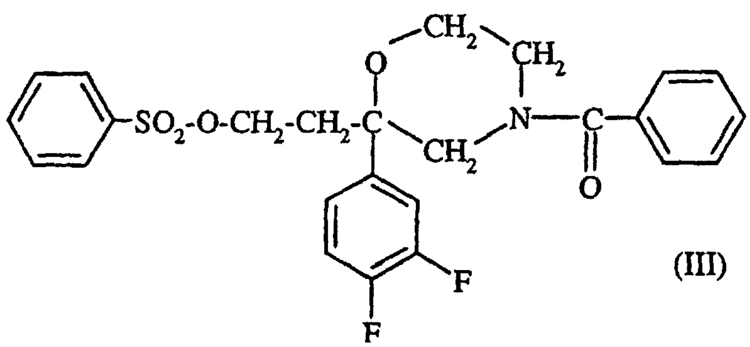

(III)

avec l'ester *tert*-butylique de l'acide (4-phénylpipéridin-4-yl)carbamique, de formule :

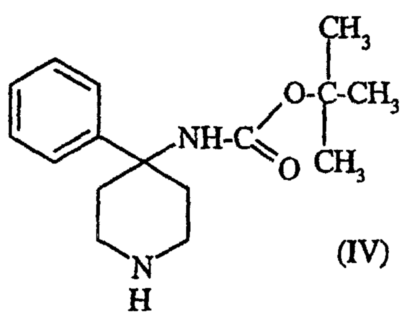

(IV)

en présence d'une base, dans un solvant inerte en mélange avec de l'eau, et éventuellement, on transforme le composé de formule (V) ainsi obtenu en un de ses sels.

**15.** Procédé selon la revendication 14 **caractérisé en ce que** le composé de formule (IV) est utilisé à raison de 1 à 1,25 équivalents molaires par équivalent molaire de composé de formule (III).

**16.** Procédé selon l'une des revendications 14 ou 15 **caractérisé en ce que** la base est un hydroxyde de métal alcalin, un carbonate ou un bicarbonate de métal alcalin.

**17.** Procédé selon l'une des revendications 14 à 16 **caractérisé en ce que** la base est utilisée à raison de 1 à 3 équivalents molaires par équivalent molaire de composé de formule (III).

**18.** Procédé selon l'une des revendications 14 à 17 **caractérisé en ce que** l'eau est utilisée à raison de 1 à 3 équivalents en volume par équivalent en poids de base.

**19.** Procédé selon l'une des revendications 14 à 18 **caractérisé en ce que** le solvant inerte est la méthylisobutylcétone, le toluène, l'acétonitrile, l'éthanol ou un mélange de ces solvants.

**20.** Procédé selon l'une des revendications 14 à 19 **caractérisé en ce que** la réaction est conduite à une température comprise entre 20°C et 90°C.

**21.** Procédé selon la revendication 1 **caractérisé en ce que** l'on prépare le (+)-[2-[2-(4-amino-4-phénylpipéridin-1-yl) éthyl]-2-(3,4-difluorophényl)morpholin-4-yl]phénylméthanone, ou l'un de ses sels, de formule :

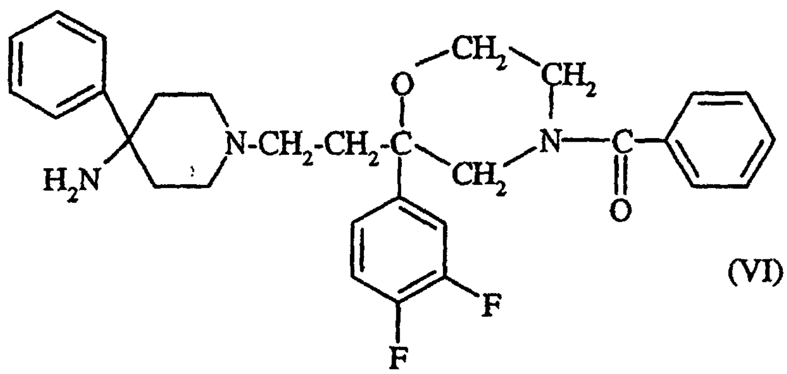

(VI)

par réaction du (+)-4-benzoyl-2-(3,4-difluorophényl)-2-(2-benzènesulfonyl oxyéthyl)morpholine de formule :

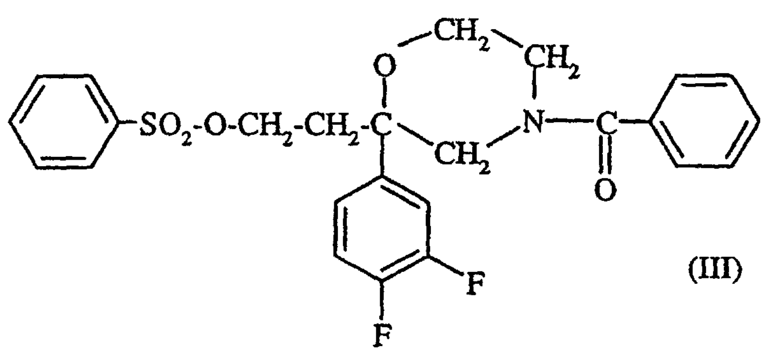

(III)

avec le 4-amino-4-phénylpipéridine de formule :

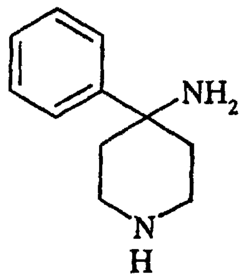

(IX)

en présence d'une base, dans un solvant inerte en mélange avec de l'eau, et éventuellement, on transforme le composé de formule (VI) ainsi obtenu en un de ses sels.

**22.** Procédé selon la revendication 21 **caractérisé en ce que** le composé de formule (IX) est utilisé à raison de 1 à 1,25 équivalents molaires par équivalent molaire de composé de formule (III).

**23.** Procédé selon l'une des revendications 21 ou 22 **caractérisé en ce que** la base est un hydroxyde de métal alcalin, un carbonate ou un bicarbonate de métal alcalin.

**24.** Procédé selon l'une des revendications 21 à 23 **caractérisé en ce que** la base est utilisée à raison de 1 à 3 équivalents molaires par équivalent molaire de composé de formule (III).

**25.** Procédé selon l'une des revendications 21 à 24 **caractérisé en ce que** l'eau est utilisée à raison de 1 à 3 équivalents en volume par équivalent en poids de base.

**26.** Procédé selon l'une des revendications 21 à 25 **caractérisé en ce que** le solvant inerte est la méthylisobutylcétone, le toluène, l'acétonitrile, l'éthanol ou un mélange de ces solvants.

**27.** Procédé selon l'une des revendications 21 à 26 **caractérisé en ce que** la réaction est conduite à une température comprise entre 20°C et 90°C.

**28.** Procédé de préparation du (+)-[2-[2-(4-amino-4-phénylpipéridin-1-yl)éthyl]-2-(3,4-difluorophényl)morpholin-4-yl] phénylméthanone, ou de l'un de ses sels, de formule :

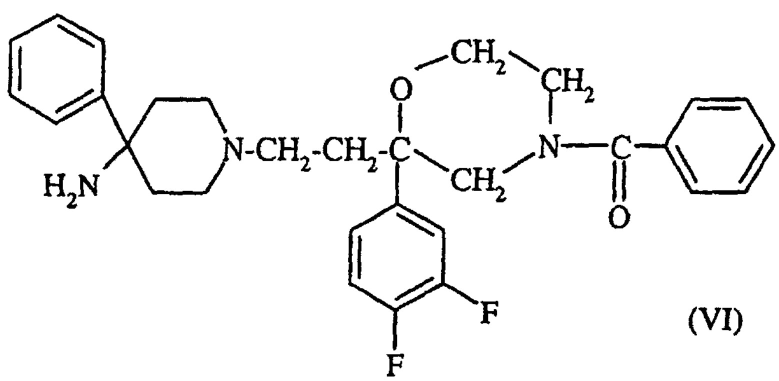

(VI)

**caractérisé en ce que** l'on déprotège l'ester tert-butylique de l'acide (+)-(1-{2-[4-benzoyl-2-(3,4-difluorophényl) morpholin-2-yl]éthyl}-4-phénylpipéridin-4-yl)carbamique, de formule :

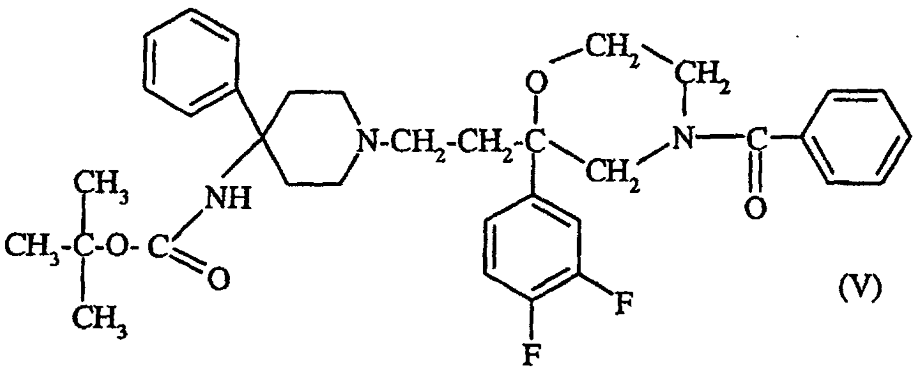

(V)

par action d'un acide, dans un solvant inerte, et, éventuellement, on transforme le composé de formule (VI) ainsi obtenu en un de ses sels.

**29.** Procédé selon la revendication 28 **caractérisé en ce que** l'on prépare l'ester *tert*-butylique de l'acide (+)-(1-{2-[4-benzoyl-2-(3,4-difluorophényl)morpholin-2-yl]éthyl}-4-phénylpipéridin-4-yl)carbamique, ou l'un de ses sels, de formule :

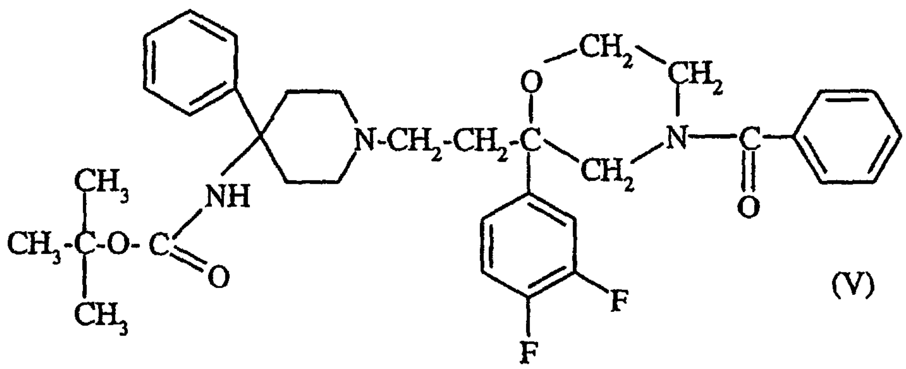

(V)

par réaction du (+)-4-benzoyl-2-(3,4-difluorophényl)-2-(2-benzènesulfonyl oxyéthyl)morpholine de formule :

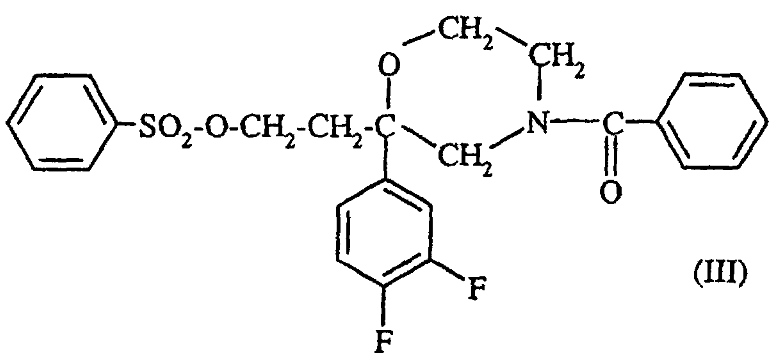

(III)

avec l'ester tert-butylique de l'acide (4-phénylpipéridin-4-yl)carbamique, de formule :

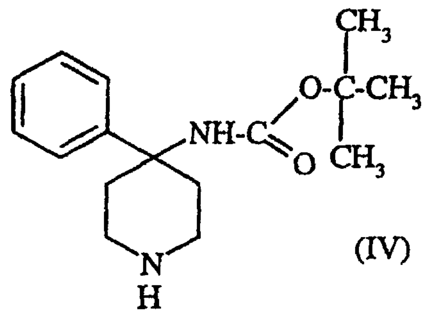

(IV)

en présence d'une base, dans un solvant inerte en mélange avec de l'eau, et éventuellement, on transforme le composé de formule (V) ainsi obtenu en un de ses sels.

**30.** Procédé de préparation de l'ester *tert*-butylique de l'acide (+)-(1-{2-[4-benzoyl-2-(3,4-difluorophényl) morpholin-2-yl]éthyl}-4-phénylpipéridin-4-yl) carbamique, ou de l'un de ses sels, de formule :

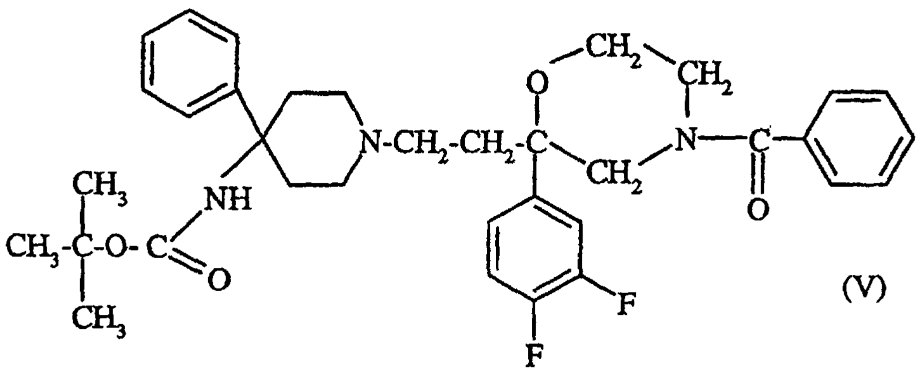

(V)

**caractérisé en ce que** l'on fait réagir le (+)-4-benzoyl-2-(3,4-difluorophényl)-2-(2-benzènesulfonyloxyéthyl) morpholine de formule :

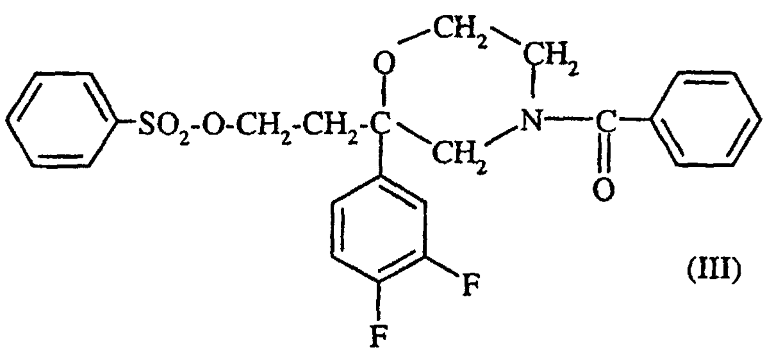

(III)

avec l'ester tert-butylique de l'acide (4-phénylpipéridin-4-yl)carbamique, de formule :

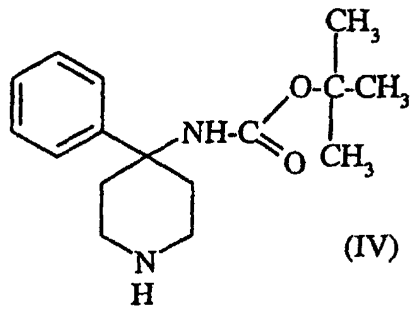

(IV)

en présence d'une base, dans un solvant inerte en mélange avec de l'eau, et éventuellement, on transforme le composé de formule (V) ainsi obtenu en un de ses sels.

**31.** Procédé de préparation du (+)-[2-[2-(4-amino-4-phénylpipéridin-1-yl)éthyl]-2-(3,4-difluorophényl)morpholin-4-yl] phénylméthanone, ou l'un de ses sels, de formule :

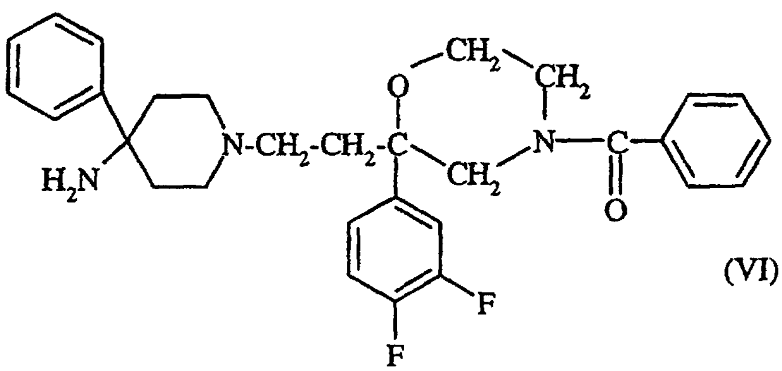

(VI)

**caractérisé en ce que** l'on fait réagir le (+)-4-benzoyl-2-(3,4-difluorophényl)-2-(2-benzènesulfonyloxyéthyl)morpholine de formule :

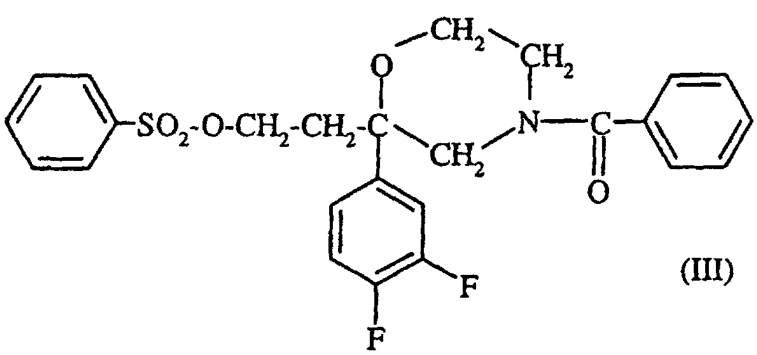

(III)

avec le 4-amino-4-phénylpipéridine de formule :

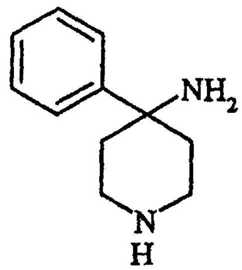

(IX)

en présence d'une base, dans un solvant inerte en mélange avec de l'eau, et éventuellement, on transforme le composé de formule (VI) ainsi obtenu en un de ses sels.

**32.** (+)-[2-[2-(4-amino-4-phénylpipéridin-1-yl)éthyl]-2-(3,4-difluorophényl) morpholin-4-yl]phénylméthanone, et ses sels, de formule :

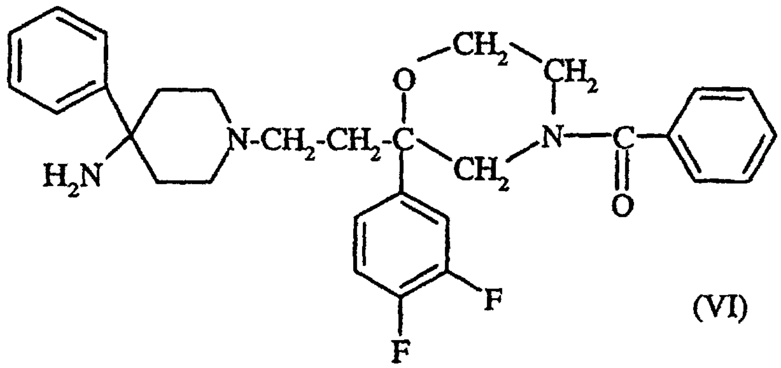

(VI)

**33.** Ester *tert*-butylique de l'acide (+)-(1-{2-[4-benzoyl-2-(3,4-difluorophényl)morpholin-2-yl]éthyl}-4-phénylpipéridin-4-yl) carbamique, et ses sels, de formule :

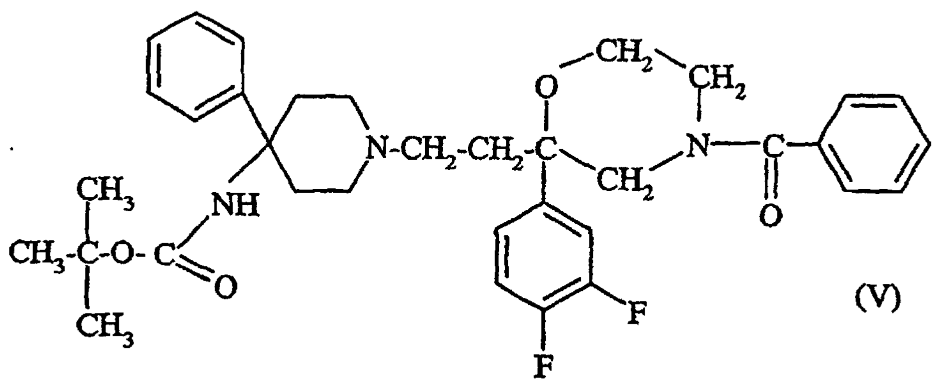

(V)

**34.** (+)-4-benzoyl-2-(3,4-difluorophinyl)-2-(2-benzène sulfonyloxyéthyl)morpholine de formule :

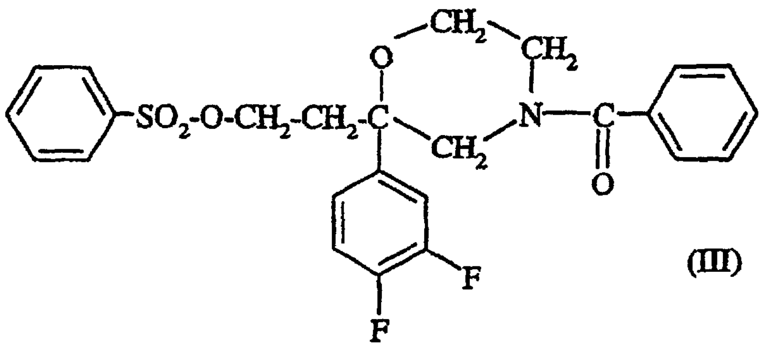

(III)

## Claims

1. Process for preparing (R)-(+)-3-{1-[2-(4-benzoyl-2-(3,4-difluorophenyl)morpholin-2-yl)ethyl]-4-phenylpiperid-4-yl}-1,1-dimethylurea, and the salts, solvates and/or hydrates thereof, of formula:

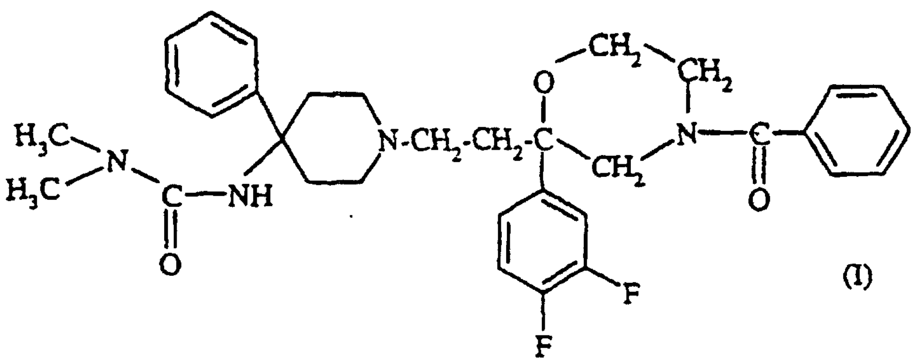

(I)

**characterized in that** (+)-[2-[2-(4-amino-4-phenylpiperid-1-yl)ethyl]-2-(3,4-difluorophenyl)morpholin-4-yl]phenylmethanone, of formula:

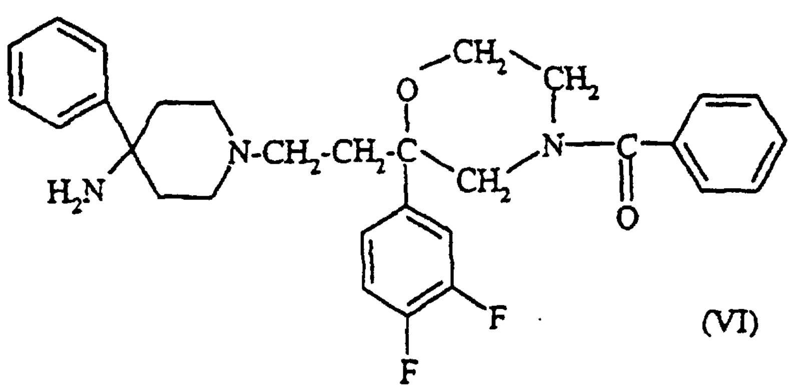

(VI)

is reacted, in an inert solvent, first with a reactive derivative of carbonic acid in the presence or absence of base, and then with dimethylamine, and the compound of formula (I) thus obtained is optionally converted into a salt thereof with pharmaceutically acceptable mineral or organic acids.

2. Process according to Claim 1, **characterized in that** the reactive derivative of carbonic acid is chosen from 1,1'-carbonyldiimidazole, phosgene and p-nitrophenyl chloroformate.

3. Process according to either of Claims 1 and 2, **characterized in that** the reactive derivative of carbonic acid is used in a proportion of from 1 to 3 molar equivalents per molar equivalent of compound of formula (VI).

4. Process according to one of Claims 1 to 3, **characterized in that**, when phosgene or p-nitrophenyl chloroformate is used, the reaction is carried out in the presence of a base chosen from triethylamine, N,N-diisopropylethylamine or N-methylmorpholine.

5. Process according to one of Claims 1 to 4, **characterized in that** the base is used in a proportion of from 1 to 6 molar equivalents per molar equivalent of reactive derivatives of carbonic acid.

6. Process according to one of Claims 1 to 5, **characterized in that** the dimethylamine is used in a proportion of from 1 to 6 molar equivalents per molar equivalent of compound of formula (VI).

7. Process according to one of Claims 1 to 6, **characterized in that** the inert solvent is dichloromethane, 1,2-dichloroethane; chloroform or carbon tetrachloride.

8. Process according to one of Claims 1 to 7, **characterized in that** the reaction is carried out at a temperature of between -20°C and 25°C.

9. Process according to Claim 1, **characterized in that** (+)-[2-[2-(4-amino-4-phenylpiperid-1-yl)ethyl]-2-(3,4-difluorophenyl)morpholin-4-yl]phenylmethanone, or a salt thereof, of formula :

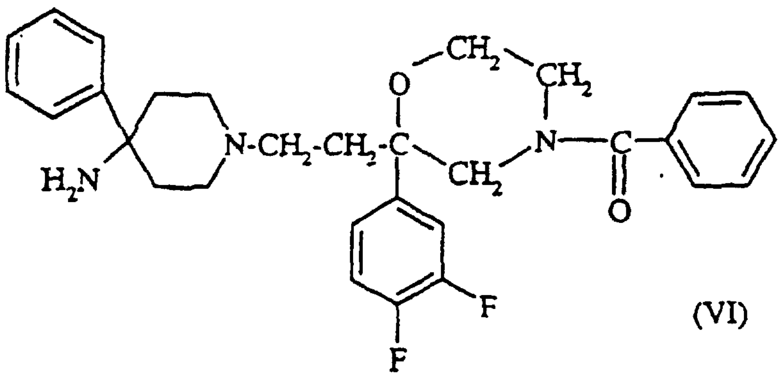

(VI)

is prepared by reacting tert-butyl (+)-(1-{2-[4-benzoyl-2-(3,4-difluorophenyl)morpholin-2-yl]ethyl}-4-phenylpiperid-4-yl)carbamate, of formula :

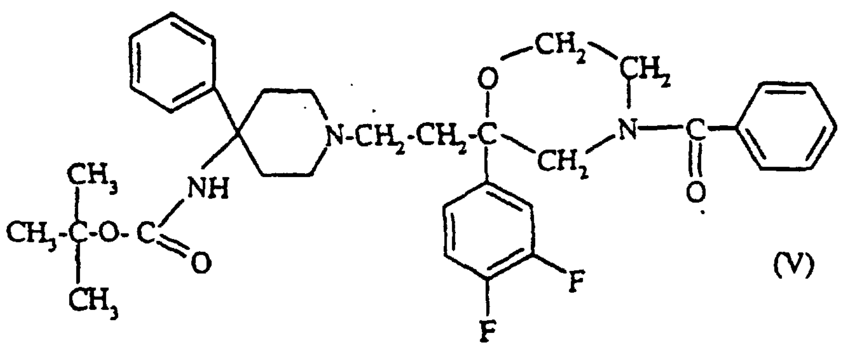

(V)

with an acid, in an inert solvent, and the compound of formula (VI) thus obtained is optionally converted into a salt thereof.

10. Process according to Claim 9, **characterized in that** the acid is hydrochloric acid, trifluoroacetic acid or formic acid.

11. Process according to either of Claims 9 and 10, **characterized in that** the acid is used in a proportion of from 4

to 10 molar equivalents per molar equivalent of compound of formula (V).

**12.** Process according to one of Claims 9 to 11, **characterized in that** the inert solvent is methyl isobutyl ketone, dichloromethane, ethyl acetate, toluene or a mixture of these solvents.

**13.** Process according to one of Claims 9 to 12, **characterized in that** the reaction is carried out at a temperature of between 10°C and 60°C.

**14.** Process according to Claim 9, **characterized in that** tert-butyl (+)-(1-{2-[4-benzoyl-2-(3,4-difluorophenyl)morpholin-2-yl]ethyl}-4-phenylpiperid-4-yl)carbamate, or a salt thereof, of formula:

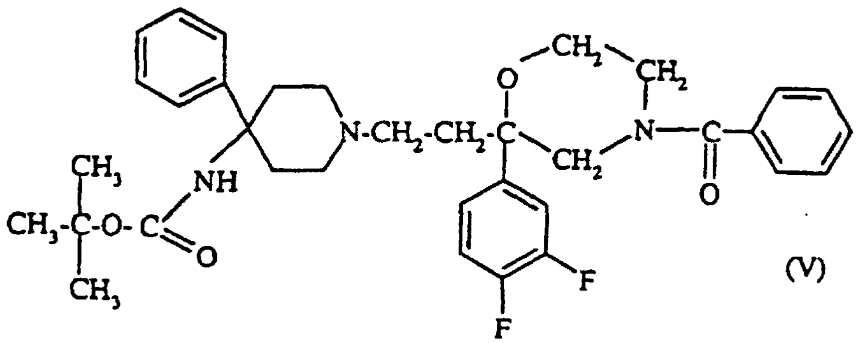

(V)

is prepared by reacting (+)-4-benzoyl-2-(3,4-difluorophenyl)-2-(2-benzenesulfonyloxyethyl)morpholine of formula:

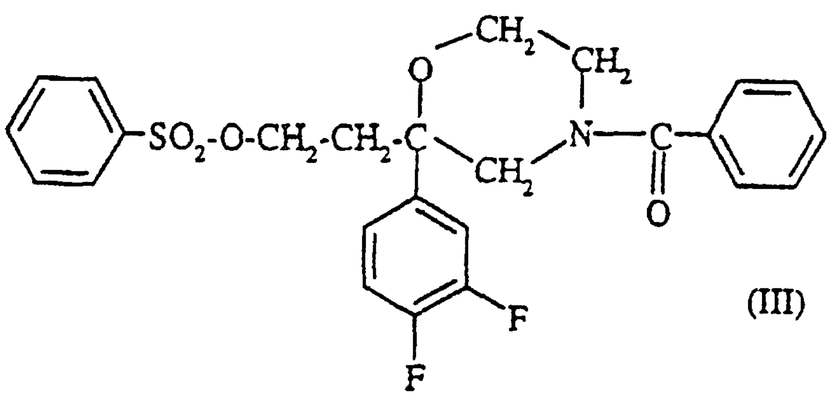

(III)

with tert-butyl (4-phenylpiperid-4-yl)carbamate, of formula:

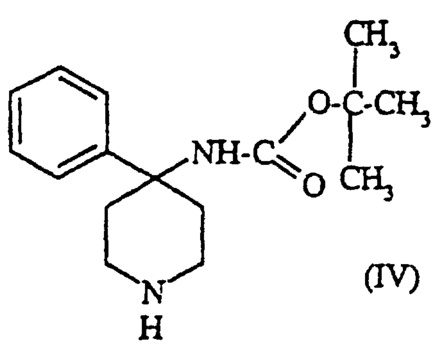

(IV)

in the presence of a base, in an inert solvent as a mixture with water, and the compound of formula (V) thus obtained

is optionally converted into a salt thereof. .

**15.** Process according to Claim 14, **characterized in that** the compound of formula (IV) is used in a proportion of from 1 to 1.25 molar equivalents per molar equivalent of compound of formula (III).

**16.** Process according to either of Claims 14 and 15, **characterized in that** the base is an alkali metal hydroxide or an alkali metal carbonate or bicarbonate.

**17.** Process according to one of Claims 14 to 16, **characterized in that** the base is used in a proportion of from 1 to 3 molar equivalents per molar equivalent of compound of formula (III).

**18.** Process according to one of Claims 14 to 17, **characterized in that** water is used in a proportion of from 1 to 3 equivalents by volume per equivalent by weight of base.

**19.** Process according to one of Claims 14 to 18, **characterized in that** the inert solvent is methyl isobutyl ketone, toluene, acetonitrile, ethanol or a mixture of these solvents.

**20.** Process according to one of Claims 14 to 19, **characterized in that** the reaction is carried out at a temperature of between 20°C and 90°C.

**21.** Process according to Claim 1, **characterized in that** (+)-[2-[2-(4-amino-4-phenylpiperid-1-yl)ethyl]-2-(3,4-difluorophenyl)morpholin-4-yl]phenylmethanone, or a salt thereof, of formula:

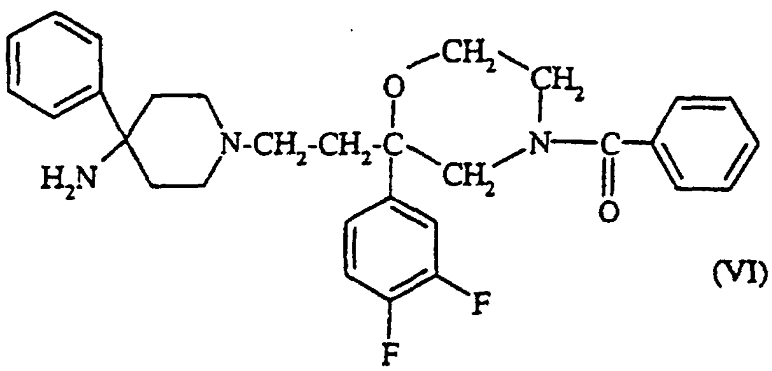

(VI)

is prepared by reacting (+)-4-benzoyl-2-(3,4-difluorophenyl)-2-(2-benzenesulfonyloxyethyl)morpholine of formula:

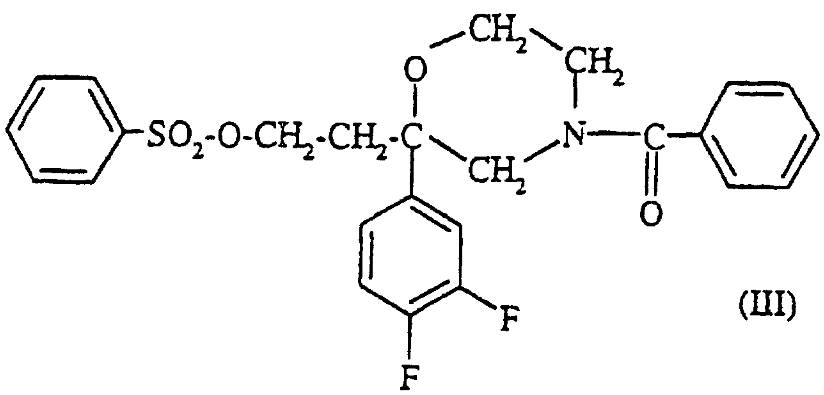

(III)

with 4-amino-4-phenylpiperidine of formula:

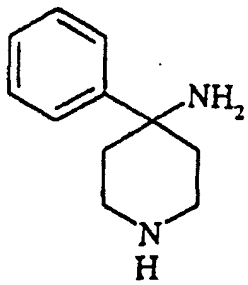

(IX)

in the presence of a base, in an inert solvent as a mixture with water, and the compound of formula (VI) thus obtained is optionally converted into a salt thereof.

**22.** Process according to Claim 21, **characterized in that** the compound of formula (IX) is used in a proportion of from 1 to 1.25 molar equivalents per molar equivalent of compound of formula (III).

**23.** Process according to either of Claims 21 and 22, **characterized in that** the base is an alkali metal hydroxide or an alkali metal carbonate or bicarbonate.

**24.** Process according to one of Claims 21 to 23, **characterized in that** the base is used in a proportion of from 1 to 3 molar equivalents per molar equivalent of compound of formula (III).

**25.** Process according to one of Claims 21 to 24, **characterized in that** water is used in a proportion of from 1 to 3 equivalents by volume per equivalent by weight of base.

**26.** Process according to one of Claims 21 to 25, **characterized in that** the inert solvent is methyl isobutyl ketone, toluene, acetonitrile, ethanol or a mixture of these solvents.

**27.** Process according to one of Claims 21 to 26, **characterized in that** the reaction is carried out at a temperature of between 20°C and 90°C.

**28.** Process for preparing (+)-[2-[2-(4-amino-4-phenylpiperid-1-yl)ethyl]-2-(3,4-difluorophenyl)morpholin-4-yl]phenylmethanone, or a salt thereof, of formula:

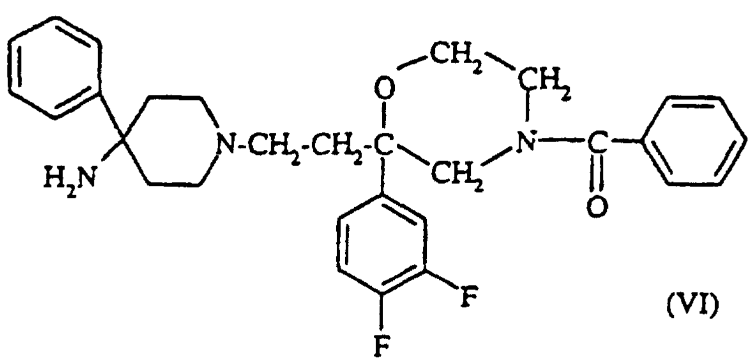

(VI)

**characterized in that** tert-butyl (+)-(1-{2-[4-benzoyl-2-(3,4-difluorophenyl)morpholin-2-yl]ethyl}-4-phenylpiperid-4-yl)carbamate, of formula:

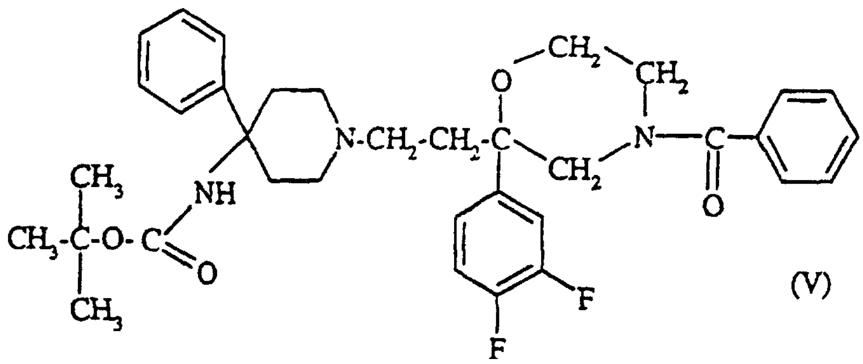

(V)

is deprotected by the action of an acid, in an inert solvent, and the compound of formula (VI) thus obtained is optionally converted into a salt thereof.

**29.** Process according to Claim 28, **characterized in that** tert-butyl (+)-(1-{2-[4-benzoyl-2-(3,4-difluorophenyl)morpholin-2-yl]ethyl}-4-phenylpiperid-4-yl)carbamate, or a salt thereof, of formula:

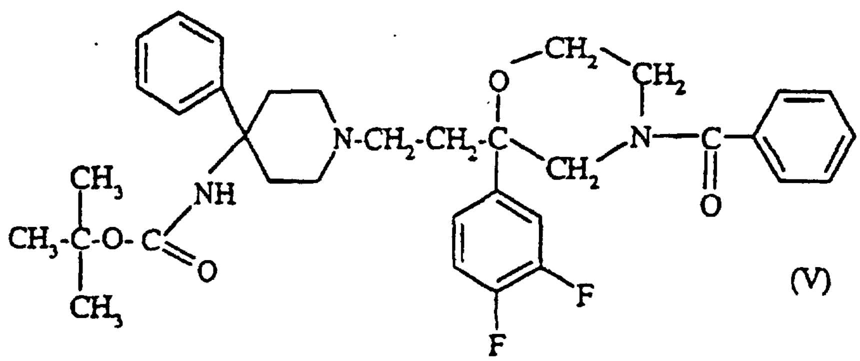

(V)

is prepared by reacting (+)-4-benzoyl-2-(3,4-difluorophenyl)-2-(2-benzenesulfonyloxyethyl)morpholine, of formula:

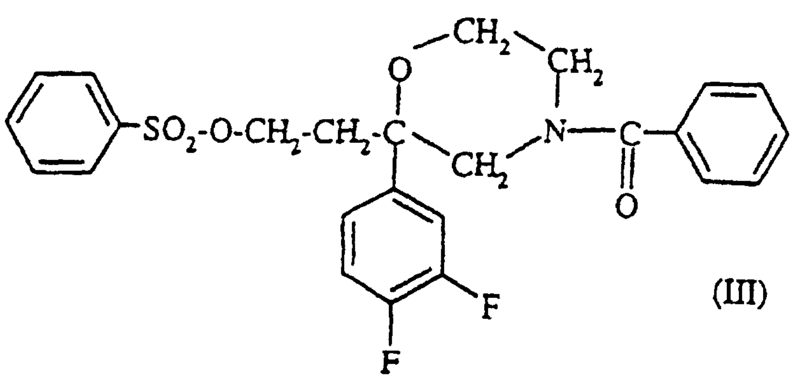

(III)

with tert-butyl (4-phenylpiperid-4-yl)carbamate, of formula:

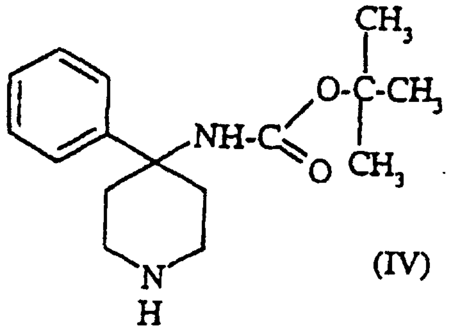

(IV)

in the presence of a base, in an inert solvent as a mixture with water, and the compound of formula (V) thus obtained is optionally converted into a salt thereof.

**30.** Process for preparing tert-butyl (+)-(1-{2-[4-benzoyl-2-(3,4-difluorophenyl)morpholin-2-yl]ethyl}-4-phenylpiperid-4-yl)carbamate, or a salt thereof, of formula:

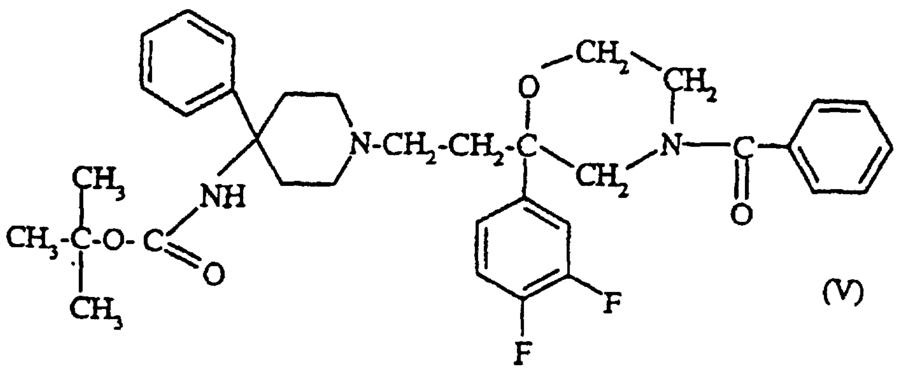

(V)

**characterized in that** (+)-4-benzoyl-2-(3,4-difluorophenyl)-2-(2-benzenesulfonyloxyethyl)morpholine, of formula:

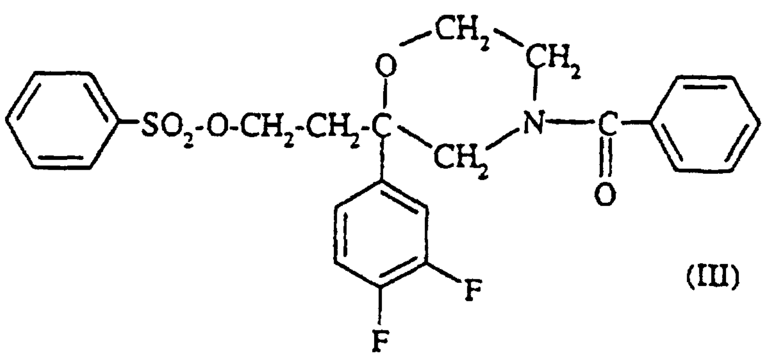

(III)

is reacted with tert-butyl (4-phenylpiperid-4-yl)carbamate, of formula:

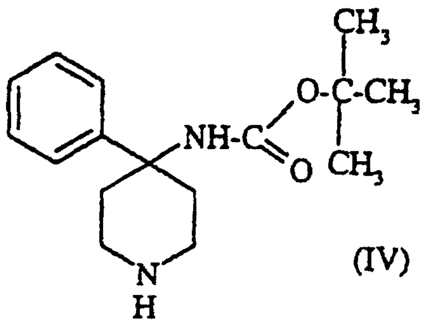

(IV)

in the presence of a base, in an inert solvent as a mixture with water, and the compound of formula (V) thus obtained is optionally converted into a salt thereof.

**31.** Process for preparing (+)-[2-[2-(4-amino-4-phenylpiperid-1-yl)ethyl]-2-(3,4-difluorophenyl)morpholin-4-yl]phenylmethanone, or a salt thereof, of formula:

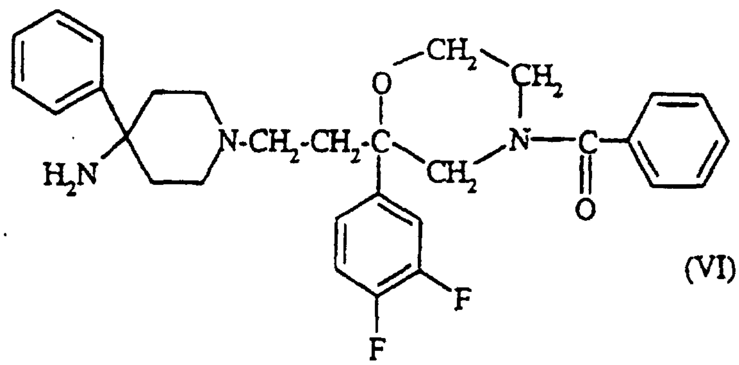

(VI)

**characterized in that** (+)-4-benzoyl-2-(3,4-difluorophenyl)-2-(2-benzenesulfonyloxyethyl)morpholine, of formula:

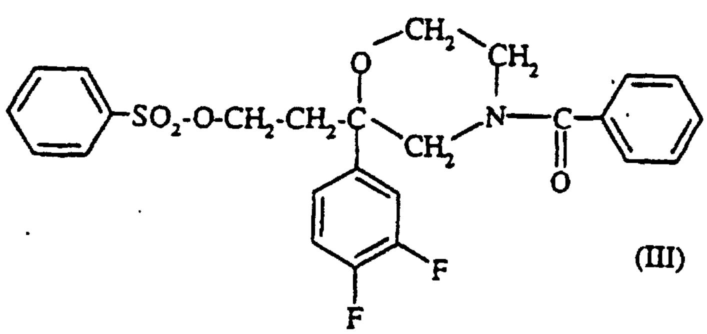

(III)

is reacted with 4-amino-4-phenylpiperidine of formula:

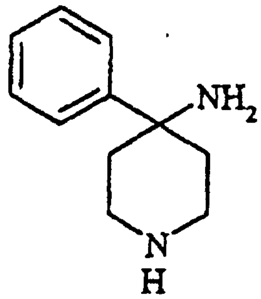

(IX)

in the presence of a base, in an inert solvent as a mixture with water, and the compound of formula (VI) thus obtained is optionally converted into a salt thereof.

**32.** (+)-[2-[2-(4-Amino-4-phenylpiperid-1-yl)ethyl]-2-(3,4-difluorophenyl)morpholin-4-yl]phenylmethanone, and the salts thereof, of formula:

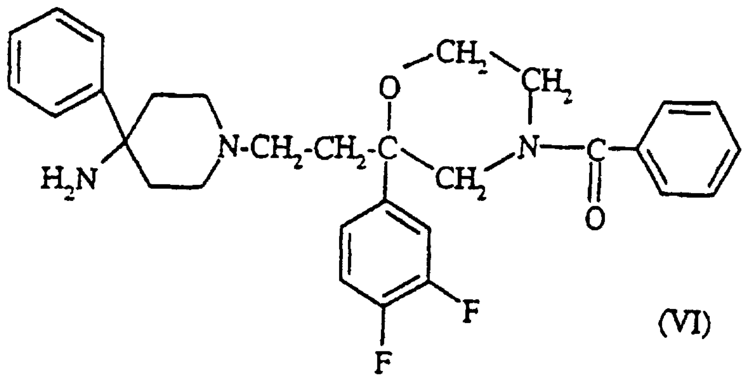

(VI)

**33.** tert-Butyl (+)-(1-{2-[4-benzoyl-2-(3,4-difluorophenyl)morpholin-2-yl]ethyl}-4-phenylpiperid-4-yl)carbamate, and the salts thereof, of formula:

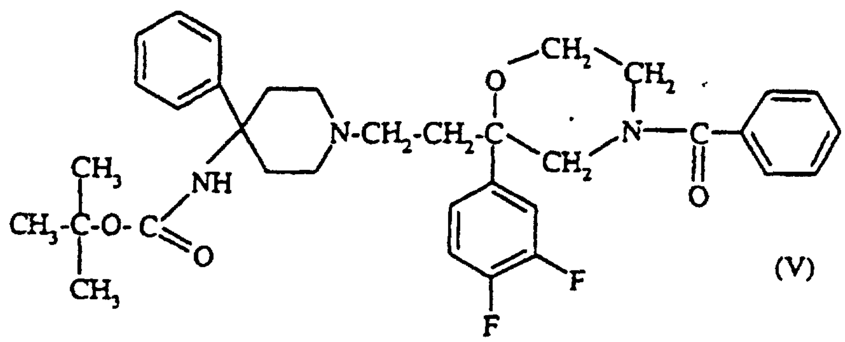

(V)

**34.** (+)-4-Benzoyl-2-(3,4-difluorophenyl)-2-(2-benzenesulfonyloxyethyl)morpholine of formula:

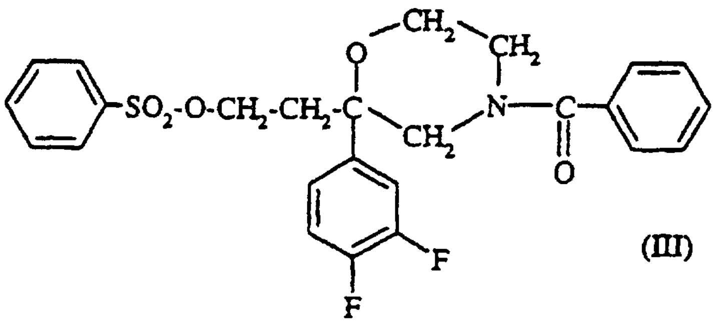

(III)

## Patentansprüche

1. Verfahren zur Herstellung von (R)-(+)-3-{1-[2-(4-Benzoyl-2-(3,4-difluorphenyl)-morpholin-2-yl)-ethyl]-4-phenylpiperidin-4-yl}-1,1-dimethylharnstoff und dessen Salze, Solvate und/oder Hydrate der Formel:

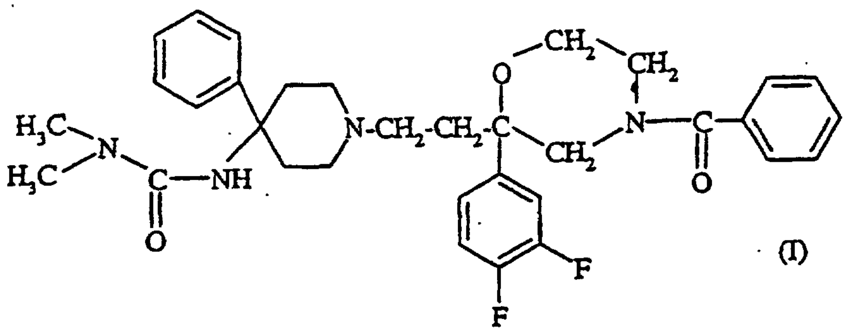

(I)

**dadurch gekennzeichnet, daß** man (+)-[2-[2-(4-Amino-4-phenylpiperidin-1-yl)-ethyl]-2-(3,4-difluorphenyl)-morpholin-4-yl]-phenylmethanon der Formel:

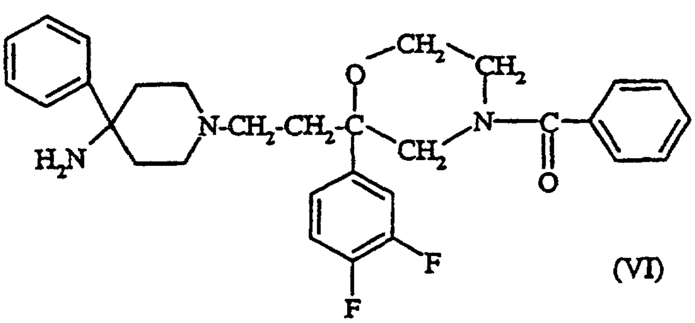

(VI)

in einem inerten Lösungsmittel zunächst mit einem reaktiven Derivat der Kohlensäure in Gegenwart oder in Abwesenheit einer Base und dann mit Dimethylamin umsetzt und gegebenenfalls die in dieser Weise erhaltene Verbindung der Formel (I) in eines ihrer Salze mit anorganischen oder organischen, pharmazeutisch annehmbaren Säuren in eines ihrer Salze umwandelt.

2. Verfahren nach Anspruch 1, **dadurch gekennzeichnet, daß** das reaktive Derivat der Kohlensäure aus 1,1'-Carbonyldiimidazol, Phosgen und Chlorameisensäure-p-nitrophenylester ausgewählt wird.

3. Verfahren nach einem der Ansprüche 1 oder 2, **dadurch gekennzeichnet, daß** das reaktive Derivat der Kohlensäure in einer Menge von 1 bis 3 Moläquivalenten pro Moläquivalent der Verbindung der Formel (VI) eingesetzt

wird.

4. Verfahren nach einem der Ansprüche 1 bis 3, **dadurch gekennzeichnet, daß** man Phosgen oder Chlorameisensäure-*p*-nitrophenylester verwendet und die Reaktion in Gegenwart einer Base ausgewählt aus Triethylamin, N, N-Diisopropylethylamin und N-Methylmorpholin durchführt.

5. Verfahren nach einem der Ansprüche 1 bis 4, **dadurch gekennzeichnet, daß** die Base in einer Menge von 1 bis 6 Moläquivalenten pro Moläquivalent der reaktiven Derivate der Kohlensäure eingesetzt wird.

6. Verfahren nach einem der Ansprüche 1 bis 5, **dadurch gekennzeichnet, daß** Dimethylamin in einer Menge von 1 bis 6 Moläquivalenten pro Moläquivalent der Verbindung der Formel (VI) eingesetzt wird.

7. Verfahren nach einem der Ansprüche 1 bis 6, **dadurch gekennzeichnet, daß** das inerte Lösungsmittel Dichlormethan, 1,2-Dichlorethan, Chloroform oder Tetrachlorkohlenstoff ist.

8. Verfahren nach einem der Ansprüche 1 bis 7, **dadurch gekennzeichnet, daß** die Reaktion bei einer Temperatur zwischen -20°C und 25°C durchgeführt wird.

9. Verfahren nach Anspruch 1, **dadurch gekennzeichnet, daß** man (+)-[2-[2-(4-Amino-4-phenylpiperidin-1-yl)-ethyl]-2-(3,4-difluorphenyl)-morpholin-4-yl]-phenylmethanon oder eines seiner Salze der Formel:

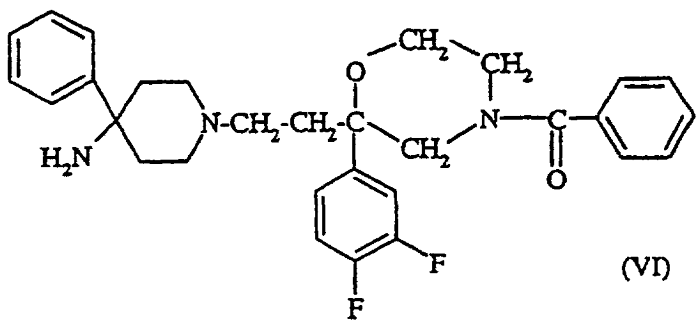

(VI)

herstellt durch Umsetzen von (+)-(1-{2-[4-Benzoyl-2-(3,4-difluorphenyl)-morpholin-2-yl]-ethyl}-4-phenylpiperidin-4-yl)-carbamidsäure-*tert.*-butylester der Formel:

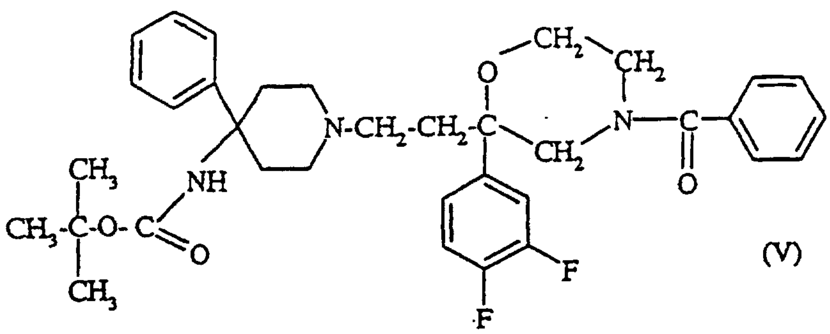

(V)

mit einer Säure in einem inerten Lösungsmittel und gegebenenfalls die in dieser Weise erhaltene Verbindung der Formel (VI) in eines ihrer Salze umwandelt.

10. Verfahren nach Anspruch 9, **dadurch gekennzeichnet, daß** man als Säure Chlorwasserstoffsäure, Trifluoressigsäure oder Ameisensäure einsetzt.

11. Verfahren nach einem der Ansprüche 9 oder 10, **dadurch gekennzeichnet, daß** die Säure in einer Menge von 4 bis 10 Moläquivalenten pro Moläquivalent der Verbindung der Formel (V) verwendet wird.

**12.** Verfahren nach einem der Ansprüche 9 bis 11, **dadurch gekennzeichnet, daß** das inerte Lösungsmittel Methylisobutylketon, Dichlormethan, Ethylacetat, Toluol oder eine Mischung dieser Lösungsmittel ist.

**13.** Verfahren nach einem der Ansprüche 9 bis 12, **dadurch gekennzeichnet, daß** die Reaktion bei einer Temperatur zwischen 10°C und 60°C durchgeführt wird.

**14.** Verfahren nach Anspruch 9, **dadurch gekennzeichnet, daß** man (+)-(1-{2-[4-Benzoyl-2-(3,4-difluorphenyl)-morpholin-2-yl]-ethyl}-4-phenylpiperidin-4-yl)-carbamidsäure-tert.-butylester oder eines seiner Salze der Formel:

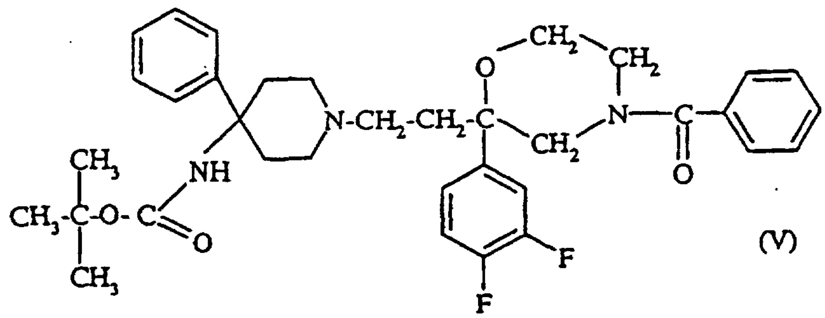

(V)

herstellt durch Reaktion von (+)-4-Benzoyl-2-(3,4-difluorphenyl)-2-(2-benzolsulfonyl-oxyethyl)-morpholin der Formel:

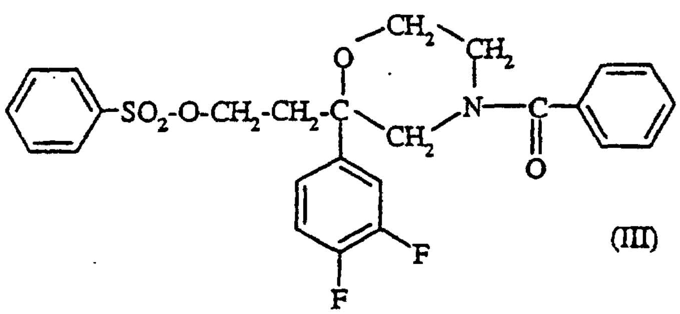

(III)

mit dem (4-Phenylpiperidin-4-yl)-carbamidsäure-*tert.*-butylester der Formel:

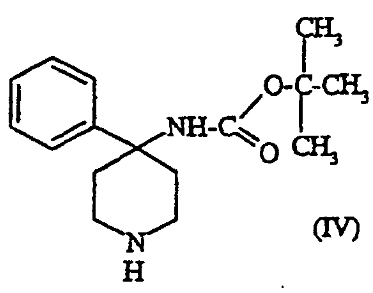

(IV)

in Gegenwart einer Base in einem inerten Lösungsmittel in Mischung mit Wasser und gegebenenfalls die in dieser Weise erhaltene Verbindung der Formel (V) in eines ihrer Salze umwandelt.

**15.** Verfahren nach Anspruch 14, **dadurch gekennzeichnet, daß** man die Verbindung der Formel (IV) in einer Menge von 1 bis 1,25 Moläquivalenten pro Moläquivalent der Verbindung der Formel (III) einsetzt.

**16.** Verfahren nach einem der Ansprüche 14 oder 15, **dadurch gekennzeichnet, daß** die Base ein Alkalimetallhydroxid, ein Alkalimetallcarbonat oder ein Alkalometallbicarbonat ist.

**17.** Verfahren nach einem der Ansprüche 14 bis 16, **dadurch gekennzeichnet, daß** die Base in einer Menge von 1 bis 3 Moläquivalenten pro Moläquivalent der Verbindung der Formel (III) eingesetzt wird.

**18.** Verfahren nach einem der Ansprüche 14 bis 17, **dadurch gekennzeichnet, daß** man Wasser in einer Menge von 1 bis 3 Volumenäquivalenten pro Gewichtsäquivalent der Base verwendet.

**19.** Verfahren nach einem der Ansprüche 14 bis 18, **dadurch gekennzeichnet, daß** das inerte Lösungsmittel Methylisobutylketon, Toluol, Acetonitril, Ethanol oder eine Mischung dieser Lösungsmittel ist.

**20.** Verfahren nach einem der Ansprüche 14 bis 19, **dadurch gekennzeichnet, daß** die Reaktion bei einer Temperatur zwischen 20°C und 90°C durchgeführt wird.

**21.** Verfahren nach Anspruch 1, **dadurch gekennzeichnet, daß** man (+)-[2-[2-(4-Amino-4-phenylpiperidin-1-yl)-ethyl]-2-(3,4-difluorphenyl)-morpholin-4-yl]-phenylmethanon oder eines seiner Salze der Formel:

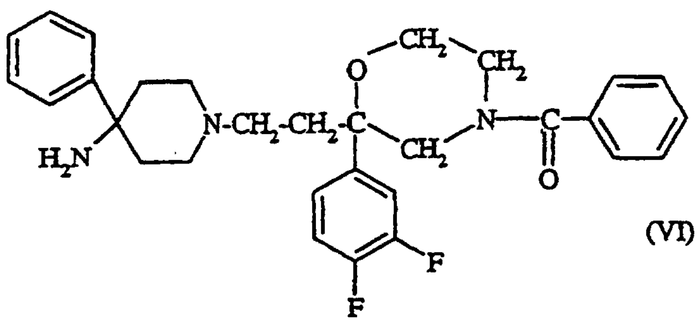

(VI)

herstellt durch Reaktion von (+)-4-Benzoyl-2-(3,4-difluorphenyl)-2-(2-benzolsulfonyl-oxyethyl)-morpholin der Formel:

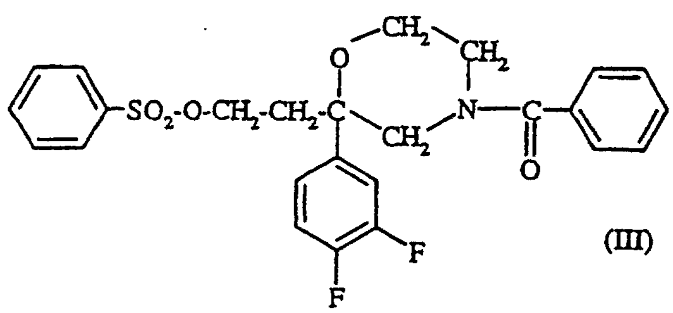

(III)

mit 4-Amino-4-phenylpiperidin der Formel:

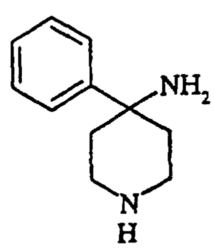

(IX)

in Gegenwart einer Base in einem inerten Lösungsmittel in Mischung mit Wasser und man gegebenenfalls die in dieser Weise erhaltene Verbindung der Formel (VI) in eines ihrer Salze umwandelt.

**22.** Verfahren nach Anspruch 21, **dadurch gekennzeichnet, daß** die Verbindung der Formel (IX) in einer Menge von

1 bis 1,25 Moläquivalenten pro Moläquivalent der Verbindung der Formel (III) eingesetzt wird.

**23.** Verfahren nach einem der Ansprüche 21 oder 22, **dadurch gekennzeichnet, daß** die Base ein Alkalimetallhydroxid, ein Alkalimetallcarbonat oder ein Alkalimetallbicarbonat ist.

**24.** Verfahren nach einem der Ansprüche 21 bis 23, **dadurch gekennzeichnet, daß** die Base in einer Menge von 1 bis 3 Moläquivalenten pro Moläquivalent der Verbindung der Formel (III) eingesetzt wird.

**25.** Verfahren nach einem der Ansprüche 21 bis 24, **dadurch gekennzeichnet, daß** man Wasser in einer Menge von 1 bis 3 Volumenäquivalent pro Gewichtsäquivalent der Base einsetzt.

**26.** Verfahren nach einem der Ansprüche 21 bis 25, **dadurch gekennzeichnet, daß** das inerte Lösungsmittel Methylisobutylketon, Toluol, Acetonitril, Ethanol oder eine Mischung dieser Lösungsmittel ist.

**27.** Verfahren nach einem der Ansprüche 21 bis 26, **dadurch gekennzeichnet, daß** die Reaktion bei einer Temperatur zwischen 20°C und 90°C durchgeführt wird.

**28.** Verfahren zur Herstellung von (+)-[2-[2-(4-Amino-4-phenylpiperidin-1-yl)-ethyl]-2-(3,4-difluorphenyl)-morpholin-4-yl]-phenylmethanon oder eines seiner Salze der Formel:

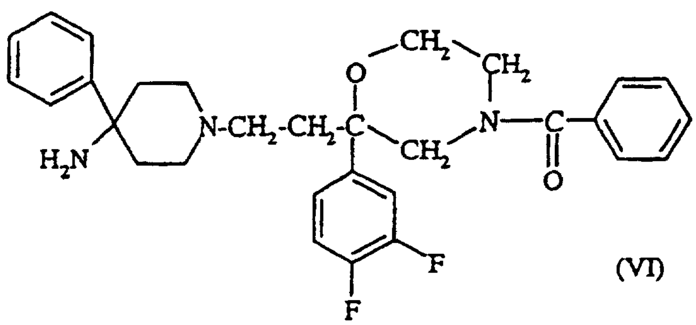

(VI)

**dadurch gekennzeichnet, daß** man die Schutzgruppe von dem (+)-(1-{2-[4-Benzoyl-2-(3,4-difluorphenyl)-morpholin-2-yl]-ethyl}-4-phenylpiperidin-4-yl)-carbamidsäure-*tert.*-butylester der Formel:

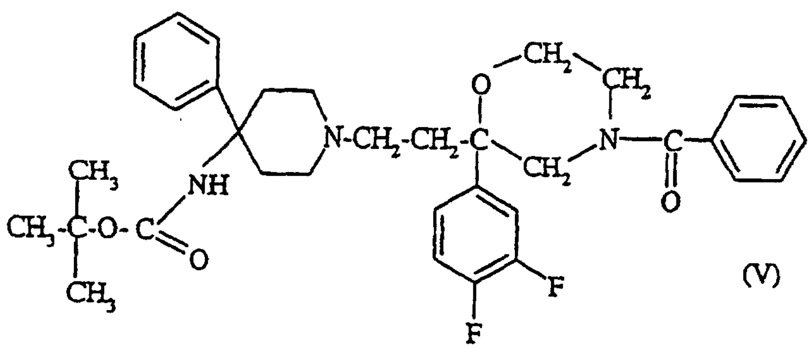

(V)

durch Einwirkung einer Säure in einem inerten Lösungsmittel abspaltet und gegebenenfalls die in dieser Weise erhaltene Verbindung der Formel (VI) in eines ihrer Salze umwandelt.

**29.** Verfahren nach Anspruch 28, **dadurch gekennzeichnet, daß** man den (+)-(1{2-[4-Benzoyl-2-(3,4-difluorphenyl)-morpholin-2-yl]-ethyl}-4-phenylpiperidin-4-yl)-carbamidsäure-*tert.*-butylester oder eines seiner Salze der Formel:

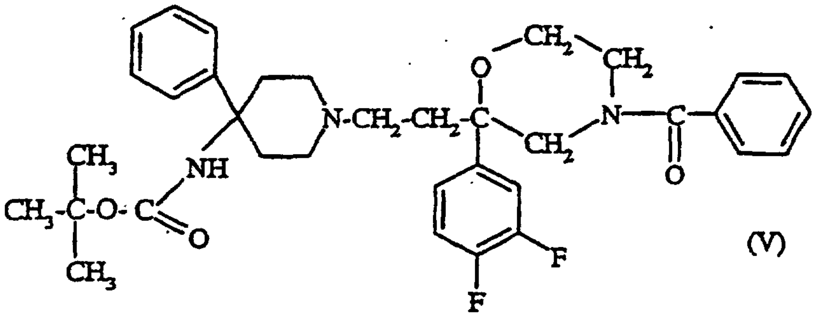

(V)

herstellt durch Reaktion von (+)-4-Benzoyl-2-(3,4-difluorphenyl)-2-(2-benzolsulfonyl-oxyethyl)-morpholin der Formel:

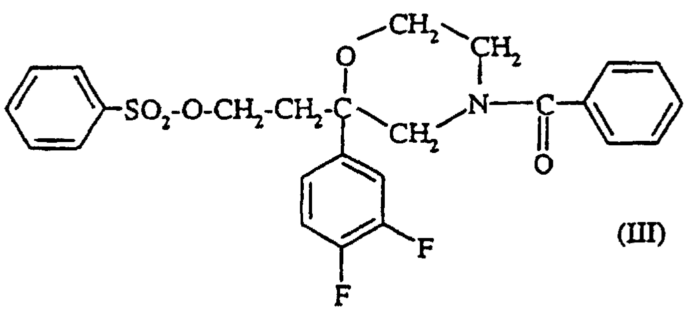

(III)

mit (4-Phenylpiperidin-4-yl)-carbamidsäure-*tert.*-butylester der Formel:

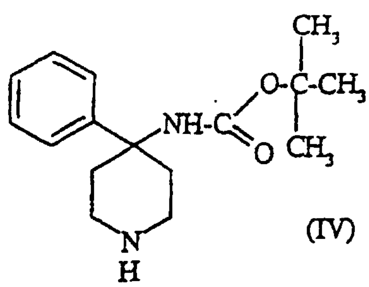

(IV)

in Gegenwart einer Base in einem inerten Lösungsmittel in Mischung mit Wasser und man gegebenenfalls die in dieser Weise erhaltene Verbindung der Formel (V) in eines ihrer Salze umwandelt.

**30.** Verfahren zur Herstellung von (+)-(1-{2-[4-Benzoyl-2-(3,4-difluorphenyl)-morpholin-2-yl]-ethyl}-4-phenylpiperidin-4-yl)-carbamidsäure-*tert.*-butylester oder eines seiner Salze der Formel:

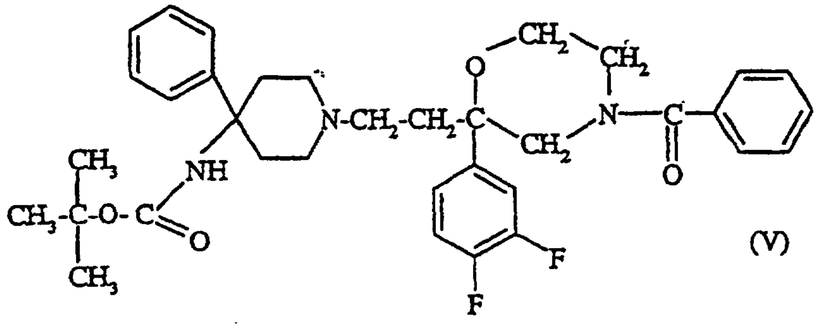

(V)

**dadurch gekennzeichnet, daß** man (+)-4-Benzoyl-2-(3,4-difluorphenyl)-2-(2-benzolsulfonyloxyethyl)-morpholin der Formel:

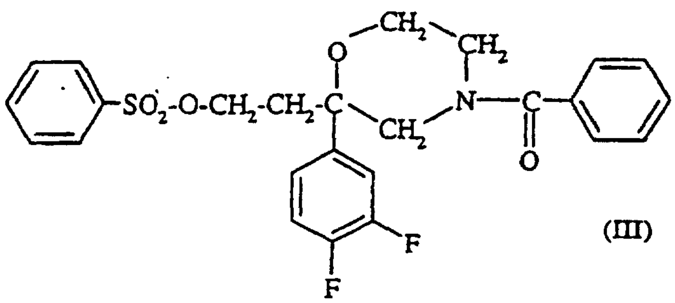

(III)

mit (4-Phenylpiperidin-4-yl)-carbamidsäure-*tert.*-butylester der Formel:

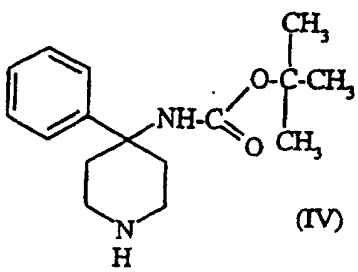

(IV)

in Gegenwart einer Base in einem inerten Lösungsmittel in Mischung mit Wasser umsetzt und gegebenenfalls die in dieser Weise erhaltene Verbindung der Formel (V) in eines ihrer Salze umwandelt.

**31.** Verfahren zur Herstellung von (+)-[2-[2-(4-Amino-4-phenylpipendin-1-yl)-ethyl]-2-(3,4-difluorphenyl)-morpholin-4-yl]-phenylmethanon oder eines seiner Salze der Formel:

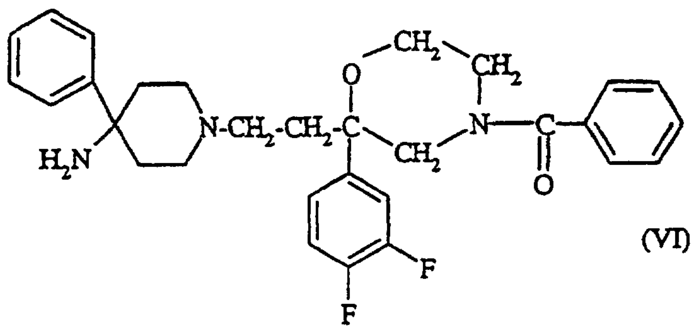

(VI)

**dadurch gekennzeichnet, daß** man (+)-4-Benzoyl-2-(3,4-difluorphenyl)-2-(2-benzolsulfonyloxyethyl)-morpholin der Formel:

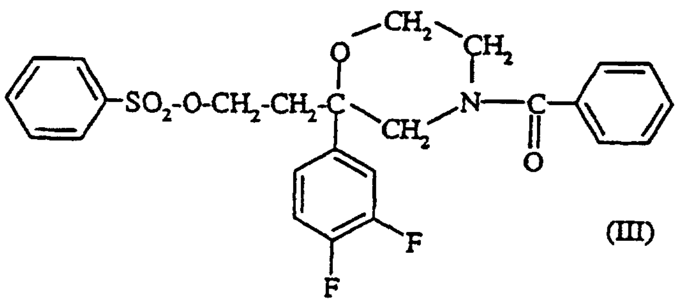

(III)

mit 4-Amino-4-phenylpiperidin der Formel:

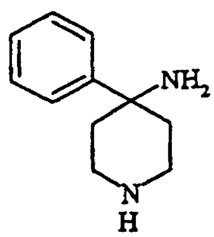

(IX)

in Gegenwart einer Base in einem inerten Lösungsmittel in Mischung mit Wasser umsetzt und gegebenenfalls die in dieser Weise erhaltene Verbindung der Formel (VI) in eines ihrer Salze umwandelt.

**32.** (+)-[2-[2-(4-Amino-4-phenylpiperidin-1-yl)-ethyl]-2-(3,4-difluorphenyl)-morpholin-4-yl]-phenylmethanon und dessen Salze der Formel:

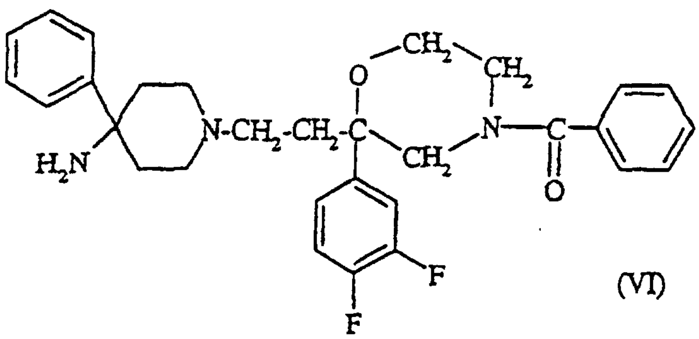

(VI)

**33.** [+)-(1-{2-[4-Benzoyl-2-(3,4-difluorphenyl)-morpholin-2-yl]-ethyl}-4-phenylpiperidin-4-yl)-carbamidsäure-*tert.*-butylester und dessen Salze der Formel:

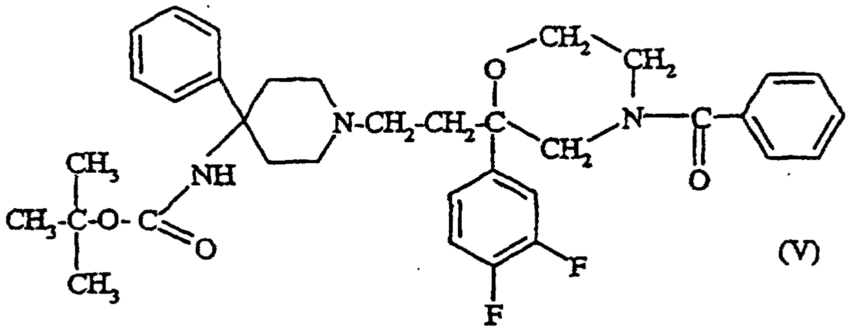

(V)

**34.** (+)-4-Benzoyl-2-(3,4-difluorphenyl)-2-(2-benzolsulfonyloxyethyl)-morpholin der Formel:

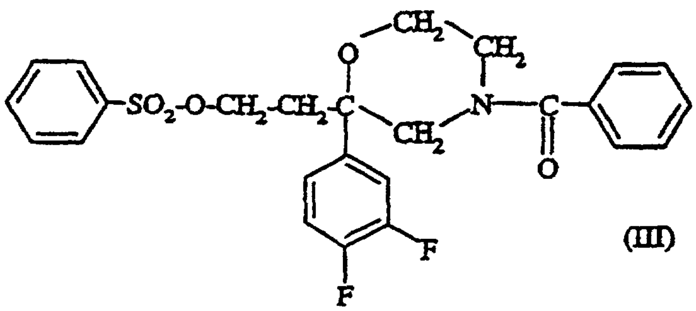

(III)